(19) Europäisches Patentamt
European Patent Office
Office européen des brevets

(11) EP 3 473 636 A1

(12) # EUROPEAN PATENT APPLICATION
published in accordance with Art. 153(4) EPC

(43) Date of publication:
**24.04.2019 Bulletin 2019/17**

(21) Application number: **17791915.6**

(22) Date of filing: **07.04.2017**

(51) Int Cl.:
***C07H 19/10** (2006.01)* ***C07H 19/20** (2006.01)*
***C07F 9/36** (2006.01)* ***C07F 9/44** (2006.01)*
***C07F 9/6561** (2006.01)* ***A61K 31/7076** (2006.01)*
***A61K 31/7072** (2006.01)* ***A61K 31/708** (2006.01)*
***A61K 31/664** (2006.01)* ***A61K 31/675** (2006.01)*
***A61P 31/20** (2006.01)* ***A61P 31/18** (2006.01)*

(86) International application number:
**PCT/RU2017/000209**

(87) International publication number:
**WO 2018/160088 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.02.2017 RU 2017106611**

(71) Applicant: **Alla Chem, LLC**
**Hallandale Beach, FL 33009 (US)**

(72) Inventors:
- **IVACHTCHENKO, Alexandr Vasilievich**
**Hallandale Beach, Florida 33009 (US)**
- **MITKIN, Oleg Dmitrievich**
**g. Khimki 141400 (RU)**

(74) Representative: **Zellentin & Partner mbB**
**Patentanwälte**
**Rubensstrasse 30**
**67061 Ludwigshafen (DE)**

(54) **NUCLEOTIDES CONTAINING AN N-[(S)-1-CYCLOBUTOXYCARBONYL]PHOSPHORAMIDATE FRAGMENT, ANALOGS THEREOF, AND USE THEREOF**

(57) The present invention relates to a prodrug and application thereof in the treatment of viral and cancerous diseases. Said prodrug inhibits HCV NS5B of HBV polymerase, DNA polymerase, and HIV-1 of reverse transcriptase (RT) and is used for the treatment of hepatitis B and C infection in mammals.

The present invention also relates to the prodrugs of general formula 1 and stereoisomers thereof and their isotopically enriched analogs, pharmaceutically acceptable salts, hydrates, solvates, or crystalline or polycrystalline forms of the prodrugs of general formula **1** and stereoisomers thereof,

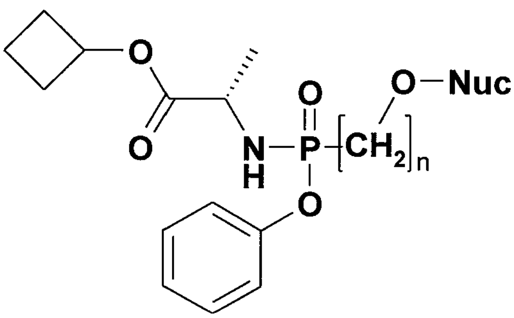

**1**

wherein **n** is 1 or 0;
**Nuc** is

EP 3 473 636 A1

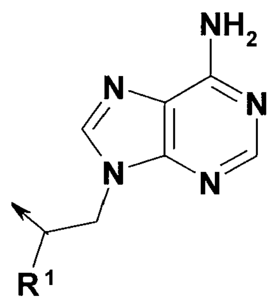

or

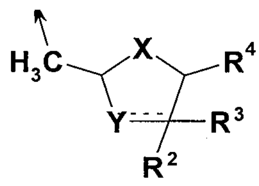

,

**R**$^1$ is hydrogen or methyl;

**R**$^2$, **R**$^3$ are optionally identical substituents selected from H, F, Cl, $CH_3$, OH provided that a solid line together with a dashed line above thereof (----) denote a carbon-carbon single bond (C-C), or **R**$^2$ and **R**$^3$ denote hydrogen provided that a solid line together with a dashed line above thereof (----) denote a carbon-carbon double bond (C=C); **X** is O, $CH_2$ or $C{=}CH_2$; Y is O, S, $CH_2$ or a HO-CH group provided that a solid line together with a dashed line above thereof (----) denote a carbon-carbon single bond (C-C), or Y is a CH group provided that a solid line together with a dashed line above thereof (----) denote a carbon-carbon double bond (C=C).

## Description

### Field of the Invention

The present invention relates to chemotherapeutic agents for the treatment of viral and cancer diseases. These compounds are prodrugs of the inhibitors of human immunodeficiency virus (HIV), polymerase hepatitis C virus (HCV), and DNA polymerase hepatitis B virus (HBV) and are intended to treat human immunodeficiency virus, hepatitis C, hepatitis B, and co-infections HIV/HCV, HIV/HBV, HIV/HCV/HBV, and HCV/HBV.

### Background of the Invention

The human immunodeficiency virus (HIV) belongs to the group of primate lentiviruses that are the etiologic agents of Acquired Immunodeficiency Syndrome (AIDS). The disease was first described in 1981, and HIV-1 was isolated by the end of 1983. Since then, AIDS has become a worldwide epidemic expanding in scope and magnitude, as HIV infections have affected different groups of population and geographic regions. Around the globe, millions of people are now infected with HIV; once infected, individuals remain infected for life. Within a decade, the overwhelming majority of HIV-infected individuals left untreated develop fatal infections as a result of HIV-induced deficiencies in the immune system. AIDS is one of the world's most important public health problems at the start of the 21$^{st}$ century. The development of highly active antiretroviral therapy (HAART) for chronic suppression of HIV replication and AIDS prevention has been a major achievement in HIV medicine [http://basicmedicalkey.com/aids-and-lentiviruses/] .

HIV continues to be a major global public health issue. In 2015, 36.7 million people worldwide were living with HIV (of these, 1.8 million were children) - a global HIV prevalence of 0.8%. The vast majority of this number live in low- and middle-income countries. In the same year, 1.1 million people died of AIDS-related illnesses. According to experts' estimates, 78 million people have become infected with HIV and 35 million have died of AIDS-related illnesses since the epidemic began. An estimated 25.5 million HIV-infected people live in Sub-Saharan Africa. The overwhelming majority of them (estimated as 19 million) live in eastern and southern Africa, which saw 46% of new HIV infections globally in 2015. Around 40% of all people living with HIV do not know that they have the virus [http://www.avert.org/global-hiv-and-aids-statistics].

The development of antiviral drugs has significantly changed the perception of HIV/AIDS from a fatal to chronic and potentially manageable disease, and the availability and administration of antiretroviral therapy (ART) has significantly reduced mortality and morbidity associated with HIV and AIDS. There is a relationship between ART and the quality of life of people living with HIV and AIDS, and several studies have reported a strong positive association between ART and improved quality of life in different domains among people living with HIV and AIDS in both developed and developing countries [https://www.ncbi.nlm.nih.gov/pmc/articles/PMC3418767/].

HIV-infected patients are living longer lives in the era of highly active antiretroviral therapy (HAART). The performance of ART therapy may extend their lifespan by up to 70-80 years. However, concomitant infection with HBV and/or HCV leads to higher morbidity and mortality rates associated with liver diseases.
Uncontrolled HIV infection accelerates the progression of HCV-induced sclerosis of the liver.

HIV/HBV coinfection is a common phenomenon. Chronic HBV infection occurs in 5-10% of HIV-infected individuals exposed to HBV at a rate 10 times higher than the general population [http://hivinsite.ucsf.edu/InSite? page=kb-05-03-04#S1X]. HIV/HCV-coinfected patients have a three-fold greater risk of cirrhosis progression or decompensated liver disease than HCV-monoinfected patients [https://aidsinfo.nih.gov/guidelines/html/1/adult-and-adolescent-arv-guidelines/26/hcv-hiv]. In a 2006 multinational cohort of more than 25000 HIV-infected persons in the United States and Europe, 14% of deaths were liver related and, of those, 66% occurred in persons with concomitant HCV infection [http://hivinsite.ucsf.edu/InSite? page=kb-00&doc=kb-05-03-05].

Monoinfection with either HBV or HCV represents one of the major causes of chronic liver diseases globally. However, in endemic areas a substantial number of patients are infected with both viruses mainly as a result of the common routes of transmission. Numerous studies have demonstrated that dually infected patients carry a greater risk of cirrhosis and hepatocellular carcinoma compared with monoinfected patients. Strikingly, approximately 60% of patients with inactive HBV infection before HCV treatment may present HBV reactivation while other HBV-infected patients experience hepatitis B surface antigen seroconversion after clearing HCV [https://www.ncbi.nlm.nih.gov/pmc/articles/PMC4367211/].

Nucleosides (and nucleotides) have already been used in clinical practice for about 50 years and have become the cornerstone for the treatment of patients with viral infections or cancer. The development of some new medicinal preparations over the last decade has demonstrated that this class of compounds still shows considerable promise.

Nucleosides and their analogs (Nuc) such as 2'-deoxy-*L*-uridine (Nuc1), 2'-deoxy-*D*-uridine (Nuc2), telbivudine (Nuc3), zidovudine (Nuc4), trifluridine (Nuc5), clevudine (Nuc6), PSI-6206 (Nuc7), 2'-(S)-2'-chloro-2'-deoxy-2'-fluorouridine (Nuc8), ND06954 (Nuc9), stavudine (Nuc10), festinavir (Nuc11), torcitabine (Nuc12), (-)-β-D-(2R,4R)-dioxolane-thymine (Nuc13), 2-(6-amino-purin-9-yl)-ethanol (Nuc14), (R)-1-(6-amino-9H-purin-9-yl)propan-2-ol (Nuc15), 2'-C-methylcytidine (Nuc16), PSI-6130 (Nuc17), gemcitabine (Nuc18), 2'-chloro-2'-deoxy-2'-fluorocytidine (Nuc19), 2',2'-dichloro-2'-deoxycytidine (Nuc20), lamivudine (Nuc21), emtricitabine (Nuc22), 2'-deoxyadenosine (Nuc23), 2'-deoxy-β-L-adenosine (Nuc24), 2'-deoxy-4'-C-ethynyl-2-fluoroadenosine (Nuc25), [(2R,4R)-4-(6-cyclopropylamino-purin-9-yl)-[1,3]dioxolan-2-yl]-methanol (Nuc26), amdoxovir (Nuc27), entecavir (Nuc28), FMCA (Nuc29), dioxolane-G (Nuc30), β-D-2'-deoxy-2'-(*R*)-fluoro-2'-β-C-methylguanosine (Nuc31), abacavir (Nuc32), didanosine (Nuc33), and others are of great interest as promising chemotherapeutic agents [M.J. Sofia. Nucleosides and Nucleotides for the treatment of viral diseases. In Annual Reports in Medicinal Chemistry 2014, Volume 49, Editor-in-Chief M.C. Desai, p 221-247. L.P. Jordheim et al. Advances in the development of nucleoside and nucleotide analogues for cancer and viral diseases. Nat. Rev. Drug. Discov. 2013 Jun;12(6), 447-464.].

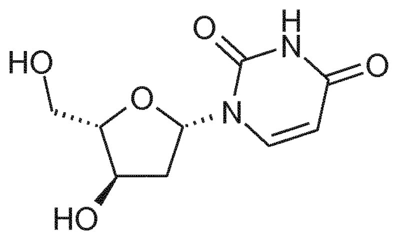

Nuc1 [CAS 31501-19-6]

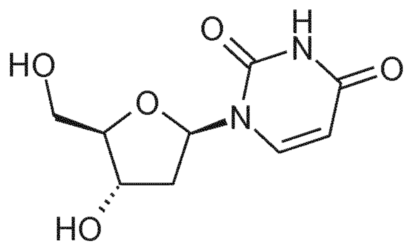

Nuc2 [CAS 951-78-0]

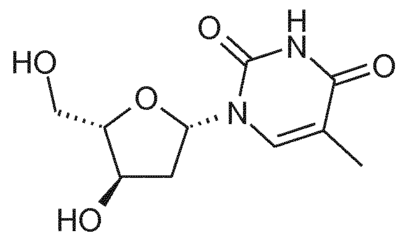

Nuc3 [CAS 3424-98-4]

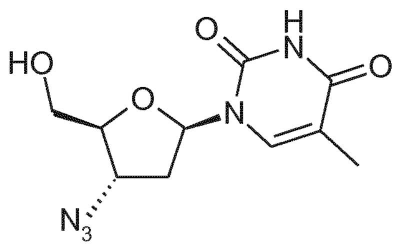

Nuc4 [CAS 30516-87-1]

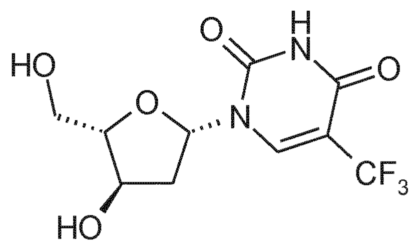

Nuc5 [CAS 70-00-8]

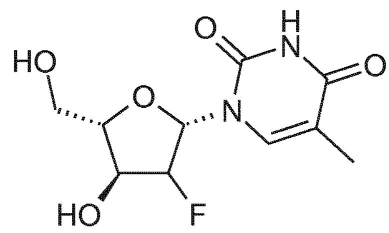

Nuc6 [CAS 163252-36-6]

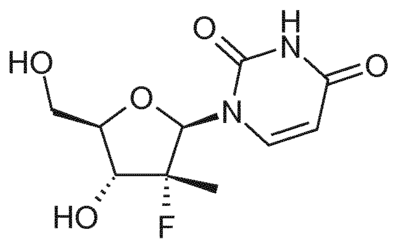

Nuc7 [CAS 863329-66-2]

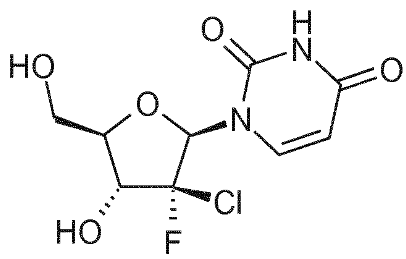

Nuc8 [CAS 1673560-41-2]

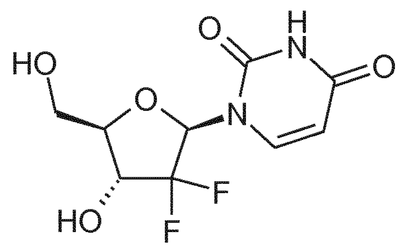

Nuc9 [CAS 114248-23-6]

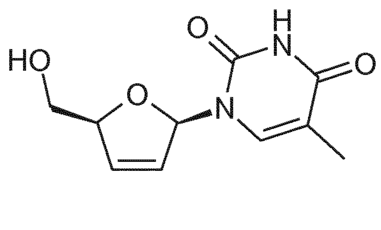

Nuc10 [CAS 3056-17-5]

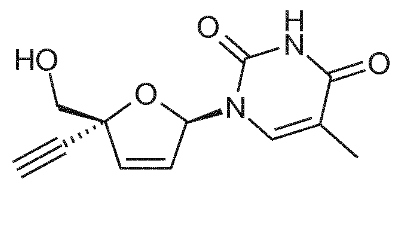

Nuc11 [CAS 634907-30-5]

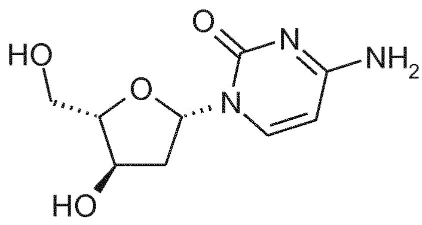

Nuc12 [CAS 40093-94-5]

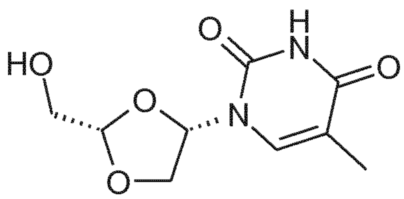

Nuc13 [CAS 127658-07-5]

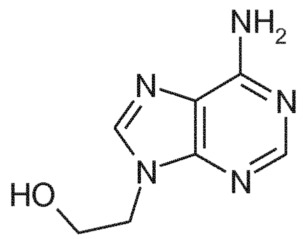

Nuc14 [CAS 07-99-3]

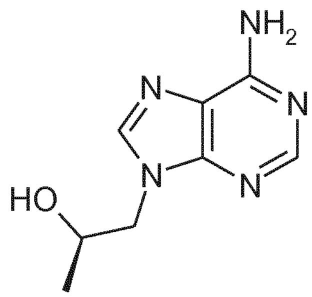

Nuc15 [CAS 14047-28-0]

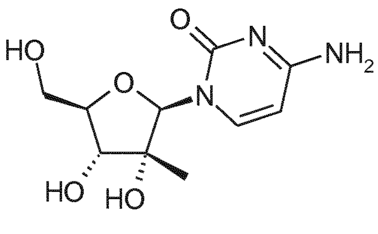

Nuc16 [CAS 20724-73-6]

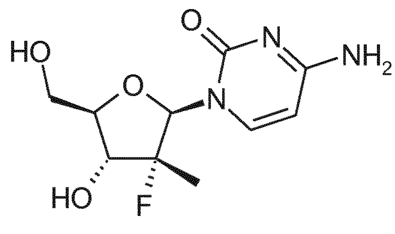

Nuc17 [CAS 817204-33-4]

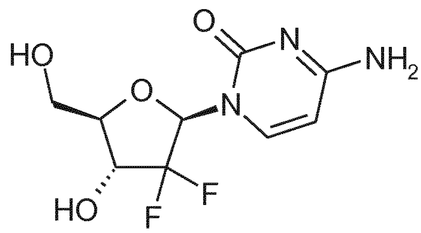

Nuc18 [CAS *95058-81-4]*

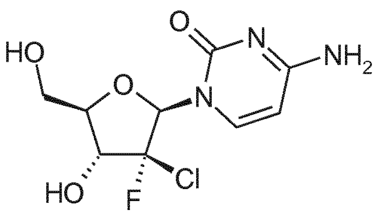

Nuc19 [CAS 1786426-19-4]

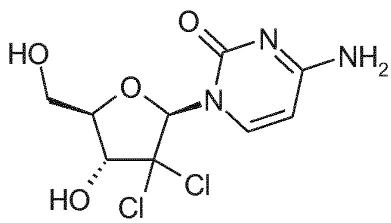

Nuc20 [CAS 1703785-65-2]

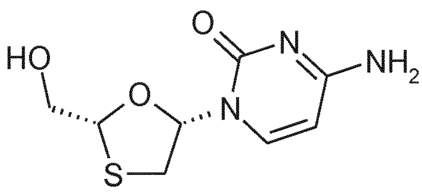

Nuc21 [CAS 134678-17-4]

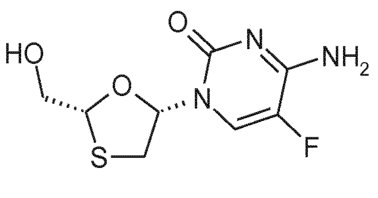

Nuc22 [CAS 143491-57-0]

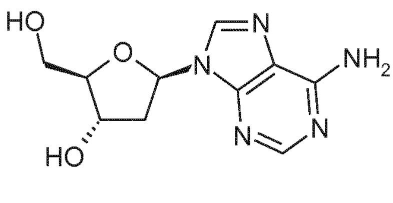

Nuc23 [CAS 958-09-8]

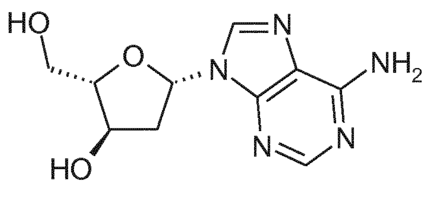

Nuc24 [CAS 14365-45-8]

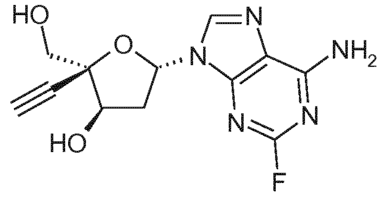

Nuc25 [CAS 865363-93-5]

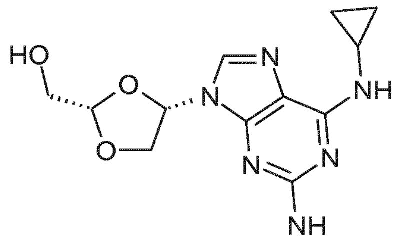

Nuc26 [CAS 1446751-04-7]

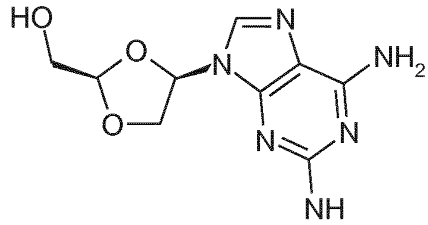

Nuc27 [CAS 145514-04-1]

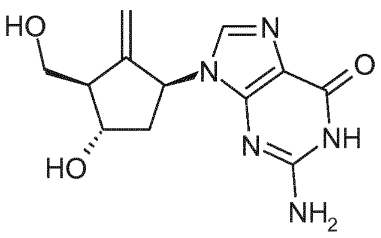

Nuc28 [CAS 209216-23-9]

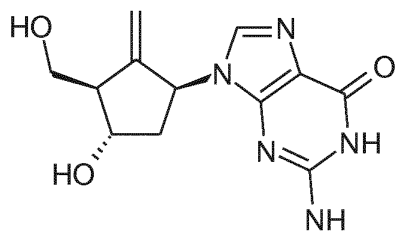

Nuc29 [CAS 1307273-70-6]

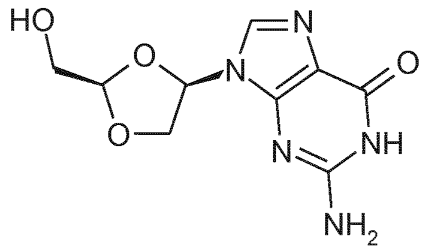

Nuc30 [CAS 145514-01-8]

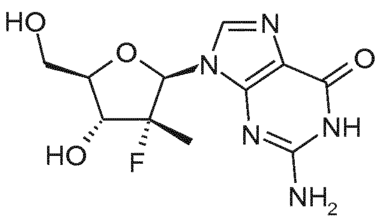

Nuc31 [CAS 817204-45-8]

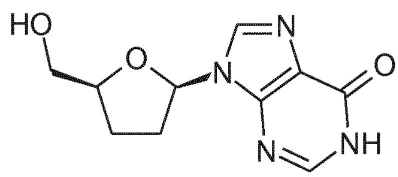

Nuc32 [CAS 136470-78-5]

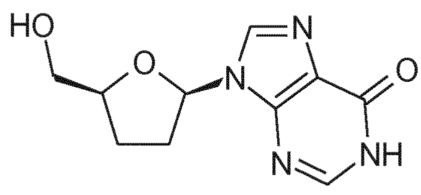

Nuc33 [CAS 69655-05-6]

Nucleos(t)ides have played an important role in the treatment of viral diseases. They appear to be the basis of some multidrug regimens for HIV-infected patients. Today, nucleos(t)ides are the preferable option and the standard of treating HBV-infected patients. They are also applied as a key component in the treatment of HCV infection. Besides, they play a principal part in the treatment of other viral infections caused by herpesviruses (HSV-1 and HSV-2), chickenpox, Epstein-Barr virus, and cytomegalovirus. [E. De Clercq, Ed. Antiviral Agents 2013, Vol. 67: Academic Press: New York. 2013. L. P. Jordheim et al. Advances in the development of nucleoside and nucleotide analogues for cancer and viral diseases. Nal. Rev. 2013, 12, 447-464.]. The attractiveness of the nucleos(t)ide strategy in the development of therapeutic agents is determined by the fact that all viruses require a polymerase either for DNA or RNA replication.

Another factor to be considered when developing a nucleos(t)ide inhibitor refers to nucleos(t)ide metabolic activation. This factor is a triphosphate nucleotide analog acting as a functional substrate for viral polymerase that is incorporated into growing RNA or DNA chains, basically leading to chain breaking and eventually to the termination of viral replication. Consequently, the efficiency of nucleos(t)ide conversion to an active triphosphate, as well as triphosphate concentration and half-life in the cell, are important factors determining the efficiency of a nucleos(t)ide as the inhibitor of viral replication. Actually, the first step of phosphorylation is critical for making triphosphate active. When the nucleoside itself is not a good substrate for kinases taking part in the initial phase of phosphorylation, it is desirable to deliver monophosphate, but this generally demands using a prodrug technology capable of masking adverse characteristics of the phosphate group and facilitating permeability. Consequently, the nucleotide prodrug strategy is very helpful when developing a nucleotide for the therapy of viral and cancer diseases.

A number of nucleos(t)ide RT inhibitors have been approved for the treatment of HIV-infection [R. F. Shinazi et al. Pharmacology of current and promising nucleosides for the treatment of human immunodeficiency viruses. J. Antiviral Res. 2006, 71, 322-334. E. De Clercq. The nucleoside reverse transcriptase inhibitors, nonnucleoside reverse transcriptase inhibitors, and protease inhibitors in the treatment of HIV infections (AIDS). Adva Pharmacol. 2013, 67, 317-358]. Combivir®, Trizivir®, Epzicom®, Truvadas, Atriple, Stribile, and Compleras. Truvadas, Atriple, Stribile, and

Compleras comprise a nucleoside, emtricitabine, and an acyclic nucleotide, tenofovir disoproxil fumarate (TDF), while CombivirTM, Trizivir, and Epzicom include a combination of two or three drugs containing nucleosides Zidovudine (AZT), Lamivudine (3TC, Nuc21, and/or Abacavir (ABC) [R. F. Shinazi et al. Pharmacology of current and promising nucleosides for the treatment of human immunodeficiency viruses. J. Antiviral Res. 2006, 71, 322-334.]. The success of antiretroviral HIV therapy has dramatically increased the lifespan of individuals infected with this disease which used to be incurable. However, despite these advances, a quest is underway to discover new agents for treating chronically infected people and reducing resistance and side effects related to long-term drug administration.

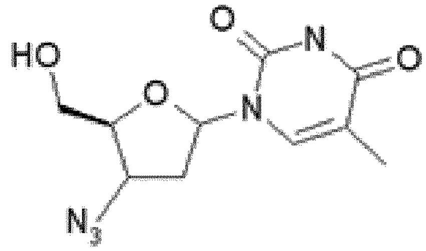

Zidovudine (AZT)

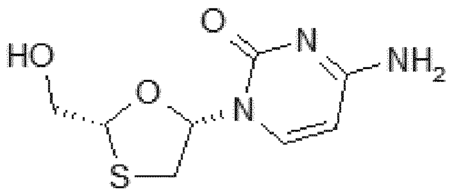

Lamivudine (3TC)

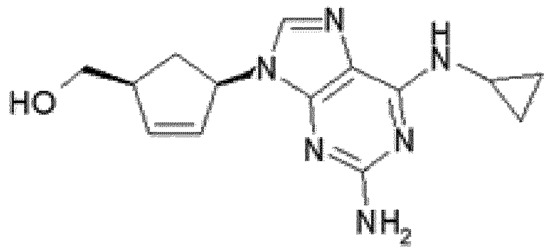

Abacavir (ABC)

One fairly new prodrug of the antiviral acyclic nucleoside tenofovir phosphonate (TFV) is ternofovir alafenamide (TAF, known as Vemlidy and GS-7340). Said prodrug possesses better properties as compared to known tenofovir disoproxil fumarate (TDF). TAF is an antiviral medication for the combination therapy of patients in need thereof. TAF was developed to increase the targeting effect of tenofovir (TFV) in the mononuclear cells of peripheral blood and, consequently, to decrease TDF-related nephrotoxicity. TAF is 400 times more effective than TFV. The use of this prodrug approach leads to a higher exposure factor of the parent nucleoside TFV in P13MCs in respect of plasma, which results in a higher efficacy [WO 2002008241. US 7390791. A.S. Ray, M.W. Fordyce, M.J.M. Hitchcock. Tenofovir alafenamide: A novel prodrug of tenofovir for the treatment of Human Immunodeficiency Virus. Antiviral Research Volume 125, January 2016, Pages 63-70. http://www.accessdata.fda.gov/drugsatfdadocs/nda/2015/ 207561Orig1s000PharmRpdf]. In 2016, the FDA approved TAF as a medicinal product for HBV treatment [https://www.hepmag.com/article/fda-approves-vemlidy-tenofovir-alafenamide-taf-hepatitis-b]. TAF is a powerful prodrug against the hepatitis B virus (HBV). As compared to TDF, TAF has less detrimental effects on bones and kidneys [http://www.aidsmap.com/Tenofovir-alafenamide-works-well-against-hepatitis-B-with-less-effect-on-bones-and-kidneys/page/3051008/].

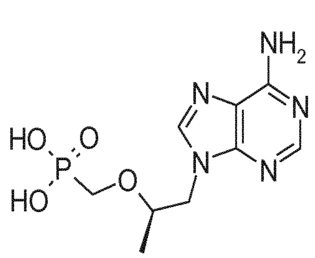

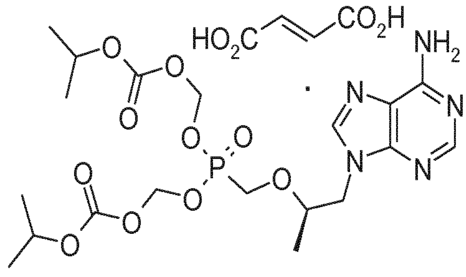

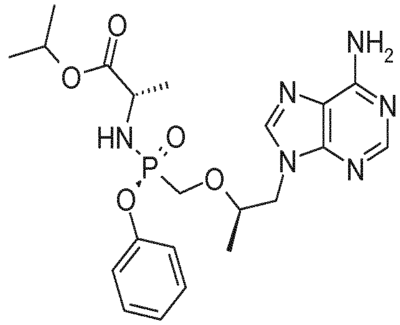

Tenofovir (TFV) Tenofovir disoproxil fumarate (TDF) Tenofovir alafenamide (TAF, GS-7340)

The properties of prodrugs for the treatment of HIV and HBV were further improved owing to tenofovir alafenamide fumarate [WO 2002008241. US 7390791] and tenofovir alafenamide hemifumarate [WO2013025788, WO 2013116720, US 9296769. http://www.gilead.ca/pdf/ca/genvoya_pm_english.pdf].

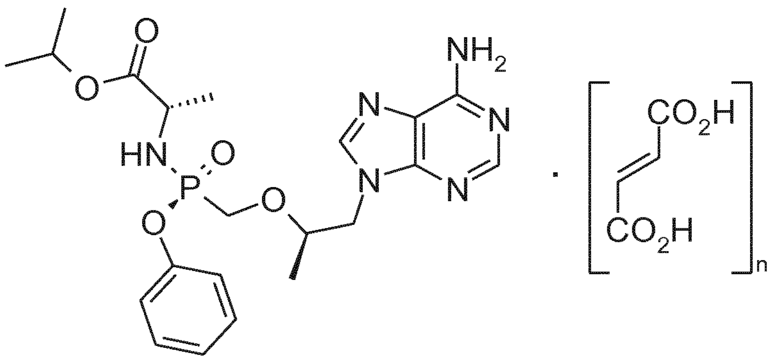

TAF fumarate: n – 1. TAF hemifumarate: n – 0.5.

Search for nucleotide phosphonates for HIV therapy that would ensure a better resistance profile against existing nucleos(t)ides and show a better safety profile against DNA polymerases has led to GS-9148. Said nucleoside phosphonate demonstrated a better resistance profile in a broad range of mutations among nucleos(t)ides and is applied in clinical practice. To improve the properties of cellular permeability and absorbance in lymphoid cells, GS-9131 was developed as a leading drug candidate. In was shown *in vivo* and *in vitro* that GS-9131, in contrast to TDF, insignificantly accumulates in the kidneys and does not show any noticeable renal toxicity [M.J. Sofia. Nucleosides and Nucleotides for the treatment of viral diseases. In Annual Reports in Medicinal Chemistry 2014, Volume 49, Editor-in-Chief M.C. Desai, p 224.]

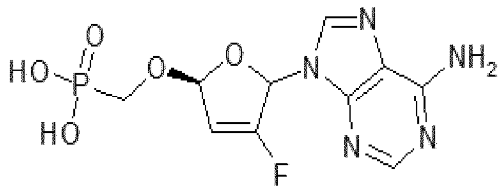

GS-9148

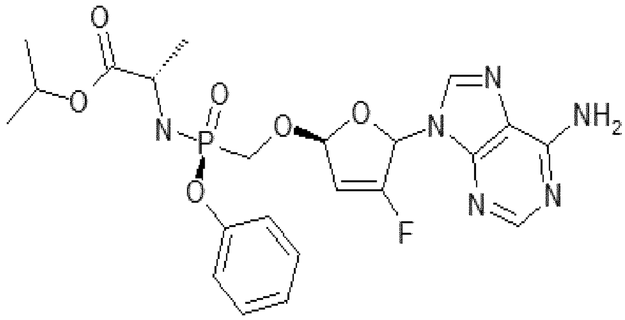

GS-9131

Among other prodrugs intended for the treatment of HIV, phosphoramidates of 6-substituted-2-H-purine dioxolanes, in particular, dioxolane-A monophosphate and dioxolane-A monophosphoramidate, were studied. The latter appeared to be the most active and the least cytotoxic with $EC_{50}$ = 0.086 μM [L. Bondada et al. Adenosine Dioxolane Nucleoside Phosphoramidates as Antiviral Agents for Human Immunodeficiency and Hepatitis B Viruses. ACS Med. Chem. Lett. 2013, 4, 747-751]

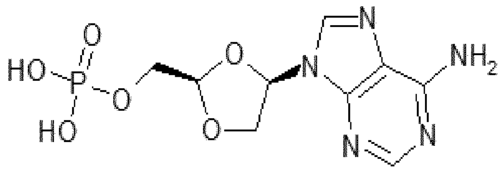

Dioxolane-A monophosphate

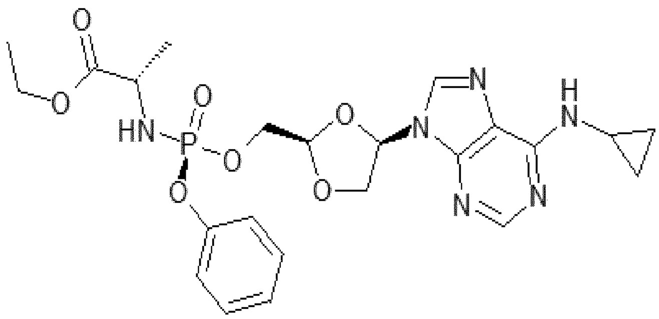

Dioxolane-A monophosphoramidate

Today, 400 million people are infected with HBV globally [http://www.pkids.org/files/pdf/ phr/02-01hbv.pdf]. The standard HBV treatment currently involves long-term nucleoside therapy. Nucleosides approved for the treatment of HBV infections include Lamivudine (3TC, Nuc21), Adefovir, Dipivoxil, Entecavir, Telbivudine, and TDF. Entecavir and TDF are the most extensively used drugs. Long-term use of Entecavir leads to resistance in a substantial population of patients, while TDF causes nephrotoxicity and bone loss [D. Grimm et al. HBV life cycle and novel drug targets. Hepatol. Int. 2011. 5. 644-653. G. Borgia, I. Gentile. Treating chronic hepatitis B: today and tomorrow. Curr. Med. Chem. 2006. 13. 2839-2855.]. Nevertheless, protracted nucleoside therapy results in the reversal of hepatic fibrosis thus demonstrating that the suppression of viral replication has a positive long-term effect [T. T. Chang et al. Long-term entecavir therapy results in the reversal of fibrosis/cirrhosis and continued histological improvement in patients with chronic hepatitis B. Hepatology 2010. 52, 886-893. P. Marcellin et al. Regression of cirrhosis during treatment with tenofovir disoproxil fumarate for chronic hepatitis B: a 5-year open-label follow-up study. Lancet 2013, 381, 468-475.].

Despite the advances of nucleos(t)ide HBV therapy, a quest is underway to discover new inhibitors showing greater advantages. Thus, some anti-HIV agents mentioned above have been studied as drugs for the treatment of HBV [C.A. Geng et al. Small-molecule inhibitors for the treatment of hepatitis B virus documented in patents. Mini Rev. Med. Chem. 2013, 13, 749-776.].

Recently, 2'-fluoro-6'-methylene-carbocyclic adenosine (FMCA) ($EC_{50}$ = 0.55 μM) has been obtained. It appeared to be a strong inhibitor of HBV replication and, in addition, demonstrated activity against Lamivudine-Entecavir resistant clone (L180M + M204V + S202G) [R.K. Rawal et al. 2'-Fluoro-6'-methylene-carbocyclic adenosine phosphoramidate (FMCAP) prodrug: In vitro anti-HBV activity against the lamivudine-entecavir resistant triple mutant and its mechanism of action. Bioorg. Med. Chem. Lett. 2013. 23, 503-506.]. Also, the production of 5'-phosphoric acid from FMCA afforded a compound 10 times more potent than FMCA against both mutants (wild, with $EC_{50}$ = 0.62 μM and resistant, with $EC_{50}$ = 0.054 μM) [R.K. Rawal et al. 2'-Fluoro-6'-methylene-carbocyclic adenosine phosphoramidate (FMCAP) prodrug: In vitro anti-HBV activity against the lamivudine-entecavir resistant triple mutant and its mechanism

of action. Bioorg. Med. Chem. Lett. 2013, 23, 503-506.].

It is also known that the prodrug clevudine (EIDD-02173) retains a high anti-HBV activity in the models of HBV infection cell cultures. The phosphoramidate moiety ensures targeted delivery of clevudine-5'-monophosphate to the liver, with the effect on other organs considerably weakened G.R. Bluemling et al. Targeted Delivery of Clevudine-5'-Monophosphate to the Liver After Oral Administration of a Clevudine-5'- Phosphoramidate Conjugate to Rats for the Treatment of HBV Infections. Global Antiviral Journal 2015, 11, Suppl. 3: HEP DART 2015: Abstr. 104, P.97].

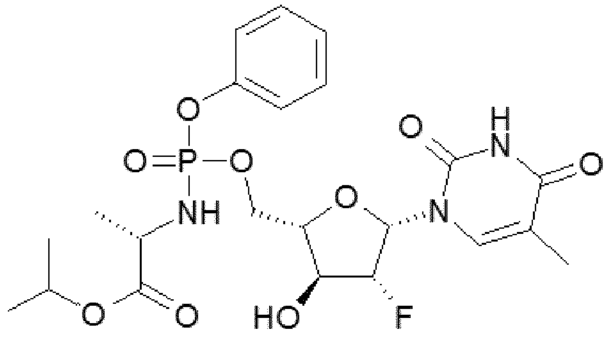

**EIDD-02173**

Gemcitabine-5'-phosphoramidate (NUC-1031) [M. Slusarczyk et al. Application of ProTide Technology to Gemcitabine: A Successful Approach to Overcome the Key Cancer Resistance Mechanisms Leads to a New Agent (NUC-1031) in Clinical Development. J. Med. Chem. 2014, 57, 1531-1542] has demonstrated a high anticancer activity. In particular, NUC-1031 appreciably reduces tumor volume *in vivo* in xenograft models of human pancreatic cancer. It is to be noted that NUC-1031 activation much less depends on nucleoside transporters and desoxycytidine than Gemcitabine. Besides, NUC-1031, as distinct from Gemcitabine, is resistant to cytidine deaminase degradation.

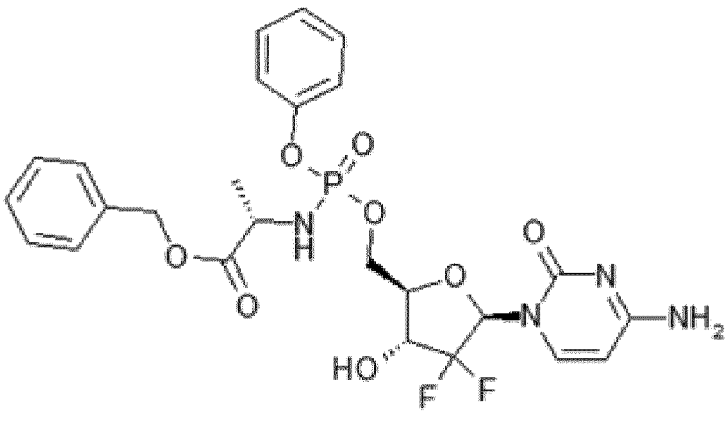

**NUC-1031**

Hepatitis C caused by HCV is one of the world's most widely spread liver diseases. According to the annual reports of the World Health Organization (WHO), more than 130-150 mln people are infected with HCV and more than 700 thousand individuals die from HCV [WHO. Hepatitis C. WHO fact sheet № 164. Updated July 2016, http://www.who.int/mediacentre /factsheets/fs164/en/]. HCV demonstrates a high genetic diversity and is characterized by regional variations of (gT) HCV genotypes. Genotype 1 (gT1) is the most common in the world (83.4 mln people, or 46.2% of all HCV-infected; about one third of them are in East Asia). Genotype 3 (gT3) is the second most common genotype. Globally, 54.3 mln people (30.1%) are infected with gT3. Genotypes 2, 4, and 6 account for 22.8% of all HCV-infected people, while genotype 5 (gT5) accounts for <1%. While genotypes 1 and 3 prevail in the majority of countries regardless of their economic status, the greatest occurrence of genotypes 4 and 5 are in low-income states [Messina, J. P. at al. Global Distribution and Prevalence of Hepatitis C Virus Genotypes. Hepatology 2015, 61(1), 77-87.].

**PSI-7851 and its diastereoisomer PSI-7977**

Recently, an inhibitor of NS5B HCV polymerase named Sofosbuvir (PSI-7851)-isopropyl (S)-2-{[(2R,3R,4R,5R)-5-(2,4-dioxo-3,4-dihydro-2H-pyrimidin-1-yl)-4-fluoro-3-hydroxy-4-methyl-tetrahydrofuran-2-ylmethoxy]-phenoxy-phosphorylamino}-propionate-and its Sp-diastereoisomer Sovaldi® (PSI-7977, GS-7977) and Rp-diastereoisomer PSI-7976 have been developed [Sofia, M. J. et al. Discovery of a β-D-2'-Deoxy-2'-a-fluoro-2'-β-C-methyluridine (Sovaldi) Nucleotide Prodrug (PSI-7977) for the Treatment of Hepatitis C Virus. J. Med. Chem. 2010, 53, 7202-7218. Sofia, M. J. et al. Nucleoside phosphoramidate prodrugs. US Patent 7964580 (2011), US Patent 8334270 (2012). RU Patent 2478104 (2013)].

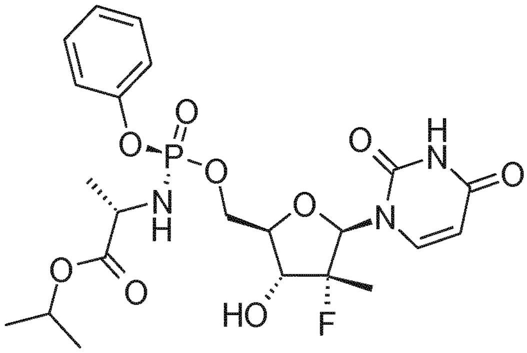

**Sovaldi® (PSI-7977)**

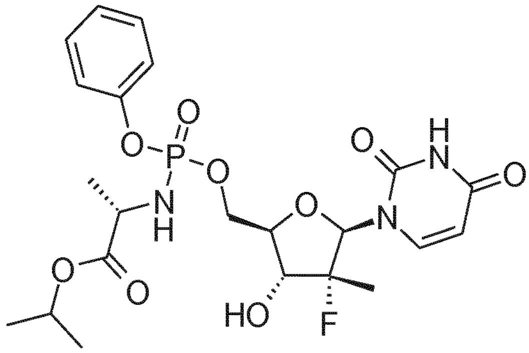

**PSI-7851**

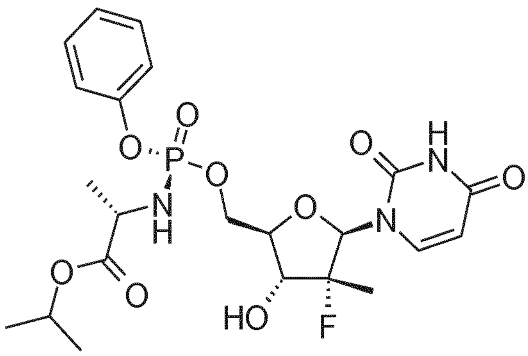

**PSI-7976**

Sovaldi® is now widely applied in the combination therapy of hepatitis C, including together with HCV NS5A inhibitors. Sovaldi® has become the first nucleotide approved by FDA and EC regulating agencies for the combination therapy of patients with hepatitis C infected with various HCV genotypes (gT). In clinical studies, it has shown high potency against six HCV genotypes (gT1-gT6) [I.M. Jacobson et al. Sofosbuvir for hepatitis C genotype 2 or 3 in patients without treatment options. Engl. J. Med. 2013, 368, 1867-1877. E. Lewirz et al. Sofosbuvir for previously untreated chronic hepatitis C infection. Engl. J. Med. 2013, 368, 1878-1887].

PSI-7851 and its diastereoisomers PSI-7976 and PSI-7977 metabolize into triphosphate PSI-7409, which actually is an HCV NS5B polymerase inhibitor [E. Murakami et al. Mechanism of activation of PSI-7851 and its diastereoisomer PSI-7977. J. Biol. Chem. 2010, 285(45), 34337-34347].

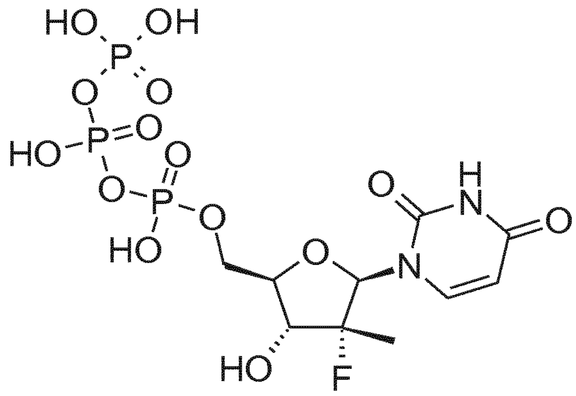

**PSI-7409**

There are other known Sovaldi® analogs [US Patent 8334270 (2012). M. J. Sofia et al. Discovery of a β-D-20-Deoxy-20-r-fluoro-20-β-C-methyluridine Nucleotide Prodrug (PSI-7977) for the Treatment of Hepatitis C Virus. J. Med. Chem. 2010, 53, 7202-7218.] including cyclohexyl (S)-2-{[(2R,3R,4R,5R)-5-(2,4-dioxo-3,4-dihydro-2H-pyrimidin-1-yl)-4-fluoro-3-hydroxy-4-methyl-tetrahydrofuran-2-ylmethoxy]-phenoxy-phosphorylamino}-propionate of formula 2.1, which, just like PSI-7851 and its phosphor stereoisomers PSI-7976 and PSI-7977 (Sovaldi®), metabolize into triphosphate PSI-7409.

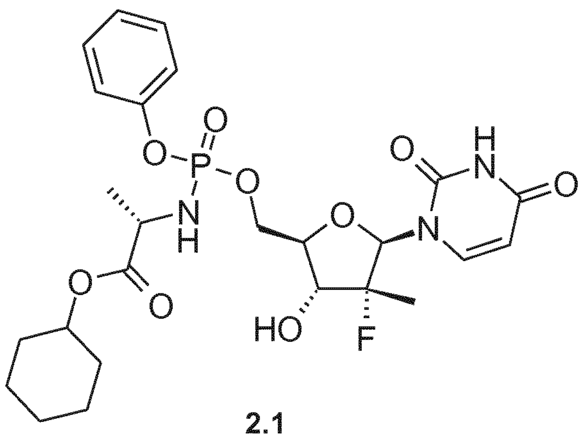

**2.1**

There are other prodrugs comprising a phosphoramidate residue for an effective hepatic transport that have been investigated. In particular, PSI-353661 has demonstrated a high inhibiting activity ($EC_{90}$ = 0.008 μM, or a more than 1000 times higher activity as compared with a guanosine analog of β-D-2'-desoxy-2'-R-fluoro-2'- β-C-methyiguanosine) and a new resistance profile similar to that of PSI-352938 [W. Clung et al. Discovery of PSI-353661, a Novel Purine Nucleotide. ACS Med. Chem. Lett. 2011. 2. 130-135.].

There are structurally related prodrugs IDX-184 ($EC_{50}$ = 0.4 μM) [X.-J. Zhou. Et al. Safety and Pharmacokinetics of IDX184, a Liver-Targeted Nucleotide Polymerase Inhibitor of Hepatitis C Virus, in Healthy Subjects Antimicrob. Agents Chegeneric. 2011, 55, 76-81. J. Lalezari, et al. Short-Term Monotherapy with IDX184, a Liver-Targeted Nucleotide Polymerase Inhibitor, in Patients with Chronic Hepatitis C Virus Infection. Antimicrob. Agents Chegeneric. 2012. 56, 6372-6378.] and INX-08189 (BMS-986094, $EC_{50}$= 0.010 μM) [C. McGuigan et al. Phosphorodiamidates as a Promising New Phosphate Prodrug Motif for Antiviral Drug Discovery: Application to Anti-HCV Agents. J. Med. Chem. 2011, 54, 8632-8645. J. H. Vernachio et al. INX-08189, a phosphoramidate prodrug of 6-O-methyl-2'-C-methyl guanosine, is a potent inhibitor of hepatitis C virus replication with excellent pharmacokinetic and pharmacodynamic properties. Antimicrob. Agents Chegeneric. 2011. 55, 1843-1851.]. These prodrugs produce a similar triphosphate but have clinically demonstrated cardiovascular toxicity associated with INX-08189 [J. J. Arnold et al. Sensitivity of Mitochondrial Transcription and Resistance of RNA Polymerase II Dependent Nuclear Transcription to Antiviral Ribonucleosides. PLOS Pathog. 2012. 8, DOI: 10.1371/journal.ppat. 1003030.]. It appears that the high cardiovascular toxicity observed in INX-08189 has diminished the interest in the development of guanosine nucleosides for the treatment of patients with HCV.

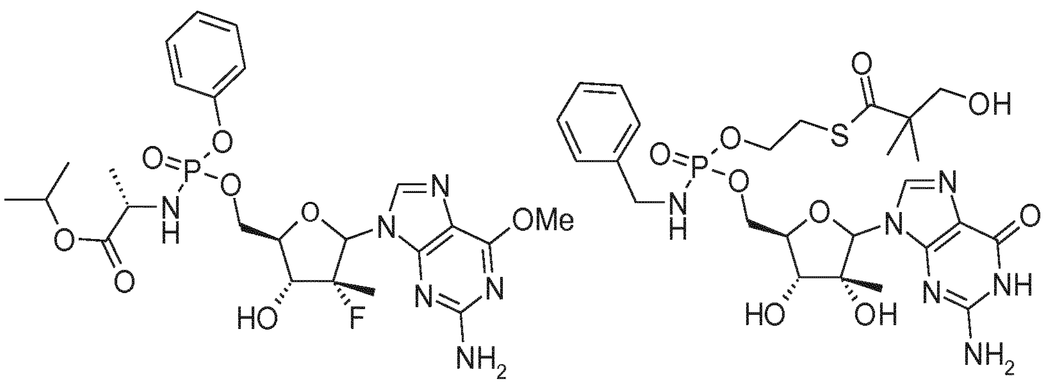

PSI-353661 IDX-184

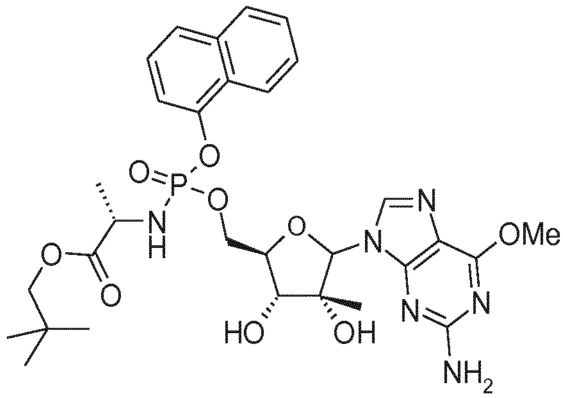

INX-08189 (BMS-986094)

It should be noted that the structure of the phosphoramidate moiety has a significant effect on the stability of phosphoramidate nucleosides in various media, on their pharmacokinetics, bioavailability, distribution in body organs, and the selectivity of their action [M. J. Sofia et al. 2010. P. Wang et al. Phosphoramidate prodrugs of (-)-β-D-(2R,4R)-dioxolane-thymine (DOT) as potent anti-HIV agents. Antiviral Chem. Chegenericapy 2012, 22, 217-238. L. Bondada et al. Adenosine Dioxolane Nucleoside Phosphoramidates as Antiviral Agents for Human Immunodeficiency and Hepatitis B Viruses. ACS Med. Chem. Lett. 2013, 4, 747-751. M. Slusarczyk et al. Application of ProTide Technology to Gemcitabine: A Successful Approach to Overcome the Key Cancer Resistance Mechanisms Leads to a New Agent (NUC-1031) in Clinical Development. J. Med. Chem. 2014, 57, 1531-1542.].

The overwhelming majority of the most effective and the least cytotoxic phosphoramidate prodrugs of practical interest are isopropyl esters (TDF, TAF, GS-9131, PSI-7851, PSI-7977, EIDD-02173), which might be explained by said esters' efficiency in targeted delivery to the nidus of infection and in metabolism into the drug.

In spite of the recent progress in antiviral therapy, the development of new prodrugs with improved characteristics and their application as chemotherapeutic agents for the treatment of viral diseases and cancer are really important.

**Summary of the invention**

The inventors have surprisingly found that previously unknown nucleotides comprising an N-[(S)-1-cyclobutoxycarbonyl]phosphoramidate moiety, their analogs of general formula 1, and stereoisomers, isotopically enriched analogs, pharmaceutically acceptable salts, hydrates, solvates, and crystalline or polycrystalline forms thereof are potent prodrugs for the treatment of viral and cancer diseases.

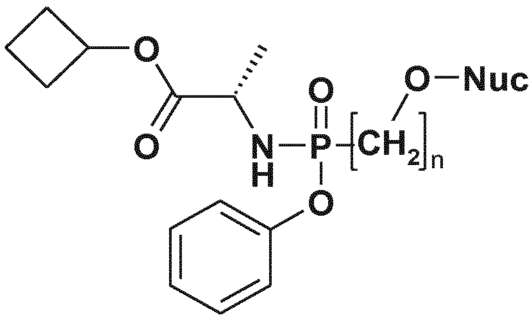

1

wherein:

n is 1 or 0;
Nuc is

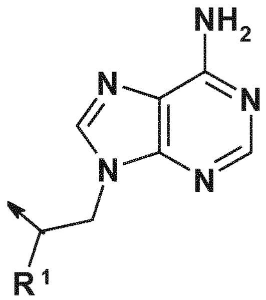

or

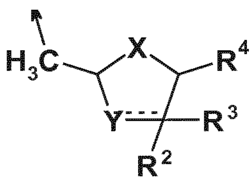

,

$R^1$ is hydrogen or methyl;
$R^2$ and $R^3$ are optionally identical substituents selected from H, F, Cl, $CH_3$, and OH provided that a solid line together with a dashed line above thereof (----) denote a carbon-carbon single (C-C) bond, or $R^2$ and $R^3$ denote hydrogen provided that the solid line together with a dashed line above thereof (----) denote a carbon-carbon double bond (C = C);
$R^4$ is a substitute selected from $R^{4.1}$- $R^{4.5}$:

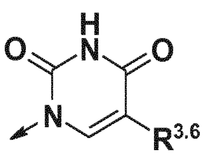

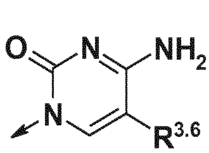

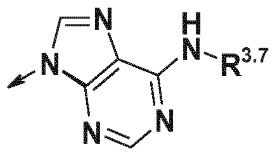

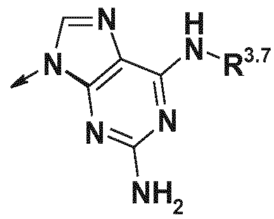

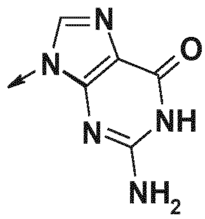

$R^{4.1}$ $R^{4.2}$ $R^{4.3}$ $R^{4.4}$ $R^{4.5}$

$R^{3.6}$ is a substitute selected from H, F, Cl, $CH_3$ or $CF_3$;
$R^{3.7}$ is hydrogen, $C_1$-$C_4$-a!ky!, or $C_3$-$C_6$-cycloalkyl;
X is O, $CH_2$, or $C=CH_2$;
Y is O, S, $CH_2$, or a HO-CH group provided that a solid line together with a dashed line above thereof (----) denote a carbon-carbon single bond (C-C), or Y is a CH group provided that a solid line together with a dashed line above thereof (----) denote a carbon-carbon single bond (C-C).

Listed below are definitions of various terms used to describe this invention. These definitions apply to the terms as they are used throughout this specification and claims, unless otherwise limited in specific instances, either individually or as part of a larger group.

The term "prodrug" refers to the compounds of this invention which have chemically or metabolically cleavable groups and become, by solvolysis or under physiological conditions, the compounds of this invention that are pharmaceutically active *in vivo*. Prodrugs often offer advantages of solubility, tissue compatibility, delivery, or delayed release in mammals (Bungard, H., Design of Prodrugs, pp. 7-9, 21-24, Elsevier, Amsterdam 1985). Prodrugs include acid derivatives well known to those skilled in the art, such as esters obtained by reaction of a starting acid compound with a suitable alcohol, or amides obtained by reaction of a starting acid compound with a suitable amine. Examples of prodrugs include, but are not limited to, acetate, formate, benzoate, and other acylated derivatives of alcohols or amines of functional groups in the compounds of this invention.

The term "cycloalkyl" refers to a monovalent saturated carbocyclic group, which can be either monocyclic or multicyclic. Representative cycloalkyl groups include, for example, cyclopropyl, cyclobutyl, cyclopentyl, cyclohexyl, cycloheptyl, cyclooctyl, and so on.

The term "active component" (drug substance) refers to a physiologically active compound of synthetic or other (biotechnological, plant, animal, bacterial, and so on) origins, which exhibits pharmacological activity and is an active ingredient of a pharmaceutical composition.

The term "medicinal drug" refers to a compound (or a mixture of compounds forming a pharmaceutical composition) in the form of tablets, capsules, injections, ointments, or other finished dosage forms intended for the restoration, improvement, or modification of physiological functions in humans and animals, and for the treatment and prophylaxis of diseases, for diagnostics, anesthesia, contraception, cosmetology, etc.

The term "therapeutic cocktail" refers to a simultaneously administered combination of two or more medicinal drugs that exhibit different mechanisms of pharmacological action and are directed at various biotargets taking part in the pathogenesis of disease.

The term "pharmaceutical composition" refers to a composition comprising the compound of general formula 1 and at least one of the components selected from the group consisting of pharmaceutically acceptable and pharmacologically compatible fillers, solvents, diluents, carriers, auxiliary, distributing, and receptive agents, excipients, delivery agents such as preservatives, stabilizers, fillers, disintegrators, moisteners, emulsifiers, suspending agents, thickeners, sweeteners, flavoring agents, aromatizing agents, antibacterial agents, fungicides, lubricants, and prolonged delivery controllers, the choice and proportions of which depend on the nature and route of administration and dosage. Examples of suitable suspending agents are ethoxylated isostearyl alcohol, polyoxyethylene, sorbitol and sorbitol ether, microcrystalline cellulose, aluminum metahydroxide, bentonite, agar-agar and tragacant, and mixtures thereof. Protection against microorganisms can be provided using various antibacterial and antifungal agents, such as parabens, chlorobutanol, sorbic acid, and the like. Said composition may also include isotonic agents, such as sugar, sodium chloride, and the like. The sustained action of the composition can be achieved using agents that decelerate the absorption of the active ingredient, for example, aluminum monostearate and gelatin. Examples of suitable carriers, solvents, diluents and delivery agents include water, ethanol, polyalcohols and mixtures thereof, natural oils (such as olive oil), and organic esters (such as ethyl oleate) for injections. Examples of fillers are lactose, milk sugar, sodium citrate, calcium carbonate, calcium phosphate, and the like. Examples of disintegrators and distributors are starch, alginic acid and salts thereof, and silicates. Examples of lubricants are magnesium stearate, sodium lauryl sulfate, talc, and polyethylene glycol of high molecular weight. A pharmaceutical composition for peroral, sublingual, transdermal, intramuscular, intravenous, subcutaneous, and local or rectal administration of the active ingredient, alone or in combination with another active compound, may be administered to animals and people in a standard administration form as a mixture with traditional pharmaceutical carriers. Suitable standard administration forms include peroral forms, such as tablets, gelatin capsules, pills, powders, granules, chewing gums, and peroral solutions or suspensions; sublingual and transbuccal administration forms; aerosols; implants; local, transdermal, subcutaneous, intramuscular, intravenous, intranasal, or intraocular forms; and rectal administration forms.

The term "inert filler" as used herein refers to a compound that is used for forming a pharmaceutical composition and is, as a rule, safe, nontoxic, and neither biologically nor otherwise undesirable, and comprises excipients acceptable for veterinary and human pharmaceutical use. Compounds of this invention may be administered individually but are generally administered in a mixture with one or more pharmaceutically acceptable excipients, diluents, or carriers chosen depending on the contemplated route of drug administration and standard pharmaceutical practice.

The term "pharmaceutically acceptable salt" refers to relatively nontoxic, both organic and inorganic salts of acids and bases claimed herein. Said salts can be obtained by *in situ* synthesis, isolation, or purification of compounds or they can be prepared specially. In particular, basic salts can be specially prepared from a purified free base of a compound claimed herein and a suitable organic or inorganic acid. Examples of salts thus prepared include hydrochlorides, hydrobromides, sulfates, bisulfates, phosphates, nitrates, acetates, dichloroacetates, oxalates, valeriates, oleates, palmitates, stearates, laurates, borates, benzoates, lactates, tosylates, citrates, maleates, fumarates, succinates, tartrates, mesylates, malonates, salicylates, propionates, ethanesulfonates, benzenesulfonates, sulfamates, and the like (a detailed description of the properties of said salts is given in Berge S.M., et al., "Pharmaceutical Salts" J. Pharm. Sci. 1977, 66: 1-19). The salts of the acids claimed herein may be also specially prepared by reaction of a purified acid with a suitable base to produce metal salts and amines. Said metal salts include the salts of sodium, potassium, calcium, barium, zinc, magnesium, lithium, and aluminum, of which sodium and potassium salts are preferable. Suitable inorganic

bases used to produce metal salts include sodium hydroxide, sodium carbonate, sodium bicarbonate, and sodium hydride; potassium hydroxide, potassium carbonate, and potassium bicarbonate; lithium hydroxide; calcium hydroxide; magnesium hydroxide; and zinc hydroxide. Suitable organic bases used to produce acid salts as claimed herein include amines and amino acids sufficiently basic to form a stable salt and suitable for medical use (in particular, they must be low-toxic). Said amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane, and the like. Furthermore, salts can be prepared using tetraalkylammonium hydroxides, such as choline, tetramethylammonium, tetraethylammonium, and the like. Amino acids may be selected from basic amino acids: lysine, ornithine, and arginine.

The term "crystalline form" refers to a substance structure wherein the molecules are arranged to form a crystal lattice.

The term "polycrystalline form" refers to a polycrystalline substance structure consisting of a plurality of monocrystals, or crystallites of certain crystallite form.

The term "therapeutically effective amount," as used herein, refers to an amount of a substance, prodrug, or drug needed for alleviating the symptoms of the disease in the subject. The dose of a substance, prodrug, or drug will meet individual demands in each particular case. Said dose may vary in a wide range depending on numerous factors like the severity of the disease to be treated, the age and the general condition of the patient, other medicaments used for the patient's treatment, the mode and route of administration, and the experience of the attending doctor. For oral administration, the daily dose is approximately 0.01-10 g, including all values therebetween, both in monotherapy and/or combination therapy. The preferred daily dose is around 0.1-7 g. As a rule, in order to alleviate or eliminate the virus, a higher loading dose is given at the beginning of treatment with a subsequent reduction of the dose to a level sufficient to prevent an infection burst.

The term "subject" refers to a mammal including, but not limited to, cattle, hogs, sheep, chickens, turkeys, buffalos, lamas, ostriches, dogs, cats, and humans; a human subject is most preferable. It is assumed that a subject's treatment may involve the use of any prodrug of general formula 1, its stereomer, isotopically enriched analog, pharmaceutically acceptable salt, hydrate, solvate, and crystalline or polymorphic form or their combinations with another compound, including with an HCV NS5A inhibitor.

The term "solvate" refers to a complex or an aggregate formed by one or more molecules of a solute, i.e., a compound of this invention or a pharmaceutically acceptable salt thereof and one or more molecules of a solvent. Said solvates are typically crystalline solids having a fixed molar ratio of the solute and the solvent. Representative solvents include, but are not limited to, water, ethanol, isopropanol, acetic acid, and so on. When the solvent is water, the solvate formed is a hydrate.

The present invention relates to a novel prodrug-a previously unknown nucleotide of general formula 1 comprising an N-[(S)-1-cyclobutoxycarbonyl]-phosphoramidate moiety, a stereoisomer thereof, and their isotopically enriched analog, pharmaceutically acceptable salt, hydrate, solvate, and crystalline or polycrystalline forms:

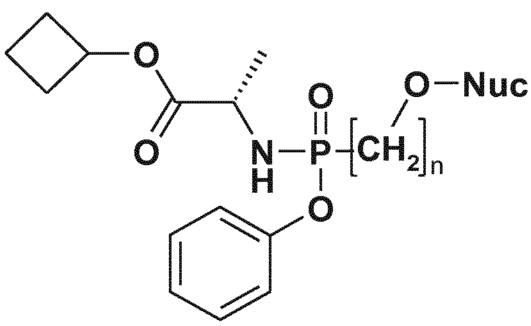

**1**

wherein

n is 1 or 0;
Nuc is

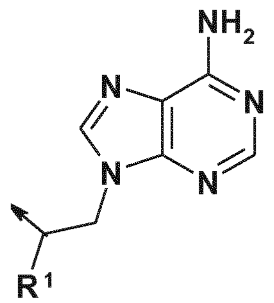

or

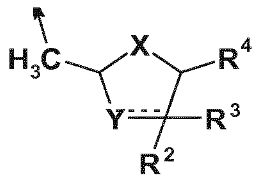

$R^1$ is hydrogen or methyl;

$R^2$ and $R^3$ are optionally identical substituents selected from H, F, Cl, $CH_3$, and OH provided that a solid line together with a dashed line above thereof (----) denote a carbon-carbon single bond (C-C), or $R^2$ and $R^3$ refer to hydrogen provided that a solid line together with a dashed line above thereof (----) denote a carbon-carbon double bond (C=C);

$R^4$ is a substitute selected from $R^{4.1}$-$R^{4.5}$:

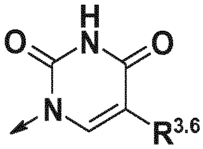

$R^{4.1}$

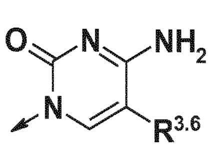

$R^{4.2}$

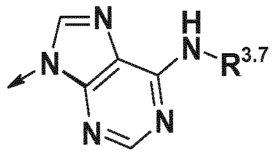

$R^{4.3}$

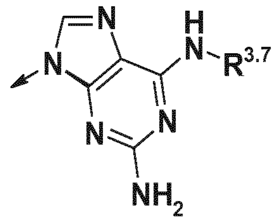

$R^{4.4}$

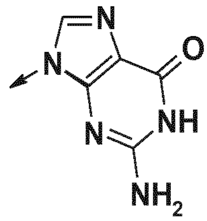

$R^{4.5}$

$R^{3.6}$ is a substitute selected from H, F, Cl, $CH_3$ or $CF_3$;

$R^{3.7}$ is hydrogen, $C_1$-$C_4$-alkyl or $C_3$-$C_6$-cycloalkyl;

X is O, $CH_2$ or C=$CH_2$;

Y is O, S, $CH_2$ or a HO-CH group provided that a solid line together with a dashed line above thereof (----) denote a carbon-carbon single bond (C-C), or Y is a CH group provided that a solid line together with a dashed line above thereof (----) denote a carbon-carbon double bond (C=C).

Preferable prodrugs are the compounds of general formulas 1A and 1B and stereoisomers thereof and their isotopically enriched analogs, pharmaceutically acceptable salts, hydrates, solvates, and the crystalline or polycrystalline forms of the compounds of general formulas 1A and 1B and stereoisomers thereof.

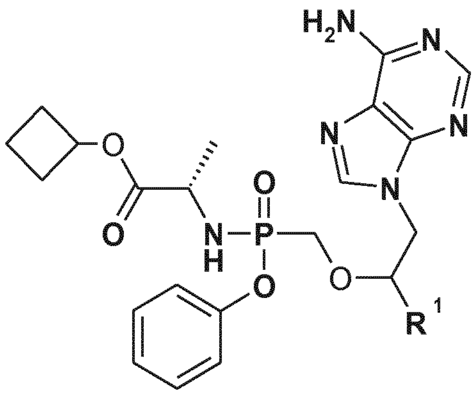

**1A**

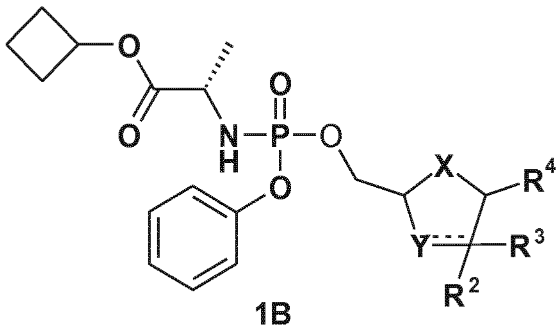

**1B**

wherein $R^1$, $R^2$, $R^3$, $R^4$, X, and Y are as defined above.

More preferable prodrugs are:

(*S*)-cyclobutyl 2-((*S*)-(((*R*)-1-(6-amino-9*H*-purin-9-yl)propan-2-yloxy)methyl) (phenoxy)phosphorylamino)-propanoate (1A.1),

(*S*)-cyclobutyl 2-((*R*)-(((*R*)-1-(6-amino-9*H*-purin-9-yl)propan-2-yloxy)methyl) (phenoxy)phosphorylamino)-propanoate (1A.2),
(*S*)-cyclobutyl 2-{(*S*)-[(2*S*,3*R*,5*S*)-3-hydroxy-5-(5-methyl-3,4-dihydro-2,4-dioxo-2H-pyrimidin-1-yl)-tetrahydrofuran-2-ylmethoxy]-phenoxy-phosphorylamino}-propanoate (1B.1),
(*S*)-cyclobutyl 2-{(*S*)-[(2*S*,3*S*,4*R*,5*S*)-3-hydroxy-4-fluoro-5-(5-methyl-3,4-dihydro-2,4-dioxo-2*H*-pyrimidin-1-yl)-tetrahydrofuran-2-ylmethoxy]-phenoxy-phosphorylamino}-propanoate (1B.2),
(*S*)-cyclobutyl 2-{(*S*)-[(2*R*,3*R*,5*R*)-5-(4-amino-2-oxo-2*H*-pyrimidin-1-yl)-3-hydroxy-4,4-difluoro-tetrahydrofuran-2-ylmethoxy]-phenoxy-phosphorylamino}-propanoate (1B.3),
(*S*)-cyclobutyl 2-{(*S*)-[(1*R*,3*S*,5*S*)-3-(2-amino-6-oxo-1,6-dihydro-purin-9-yl)-5-hydroxy-2-methylene-cyclopentylmethoxy]-phenoxy-phosphorylamino}-propanoate (1B.4),
(*S*)-cyclobutyl 2-{(*S*)-[(2*R*,5*S*)-5-(4-amino-2-oxo-2*H*-pyrimidin-1-yl)-[1,3]oxatiolan-2-ylmethoxy]-phenoxy-phosphorylamino-propanoate (1B.5),
(*S*)-cyclobutyl 2-{(*S*)-[(2*R*,5*S*)-5-(4-amino-5-fluoro-2-oxo-2*H*-pyrimidin-1-yl)-[1,3]oxatiolan-2-ylmethoxy]-phenoxy-phosphorylamino}-propanoate (1B.6),
(*S*)-cyclobutyl 2-{(*S*)-[(2*R*,3*R*,4*R*,5*R*)-5-(3,4-dihydro-2,4-dioxo-2*H*-pyrimidin-1-yl)-3-hydroxy-4-methyl-4-fluoro-tetrahydrofuran-2-ylmethoxy]-phenoxy-phosphorylamino}-propanoate (1B.7),

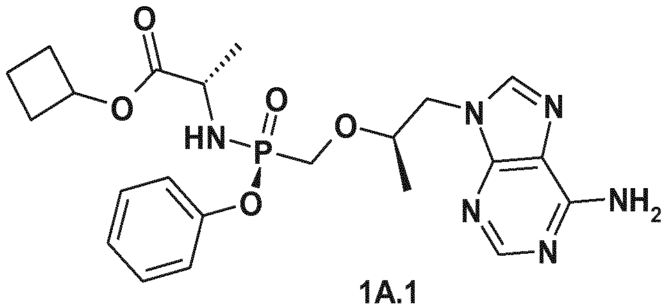

1A.1

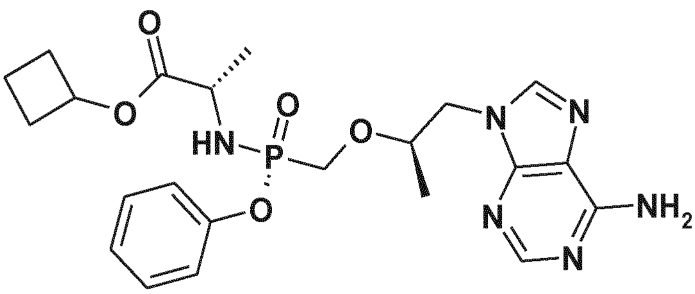

1A.2

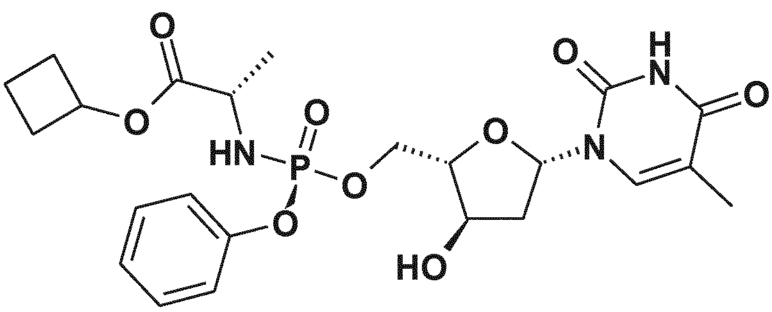

1B.1

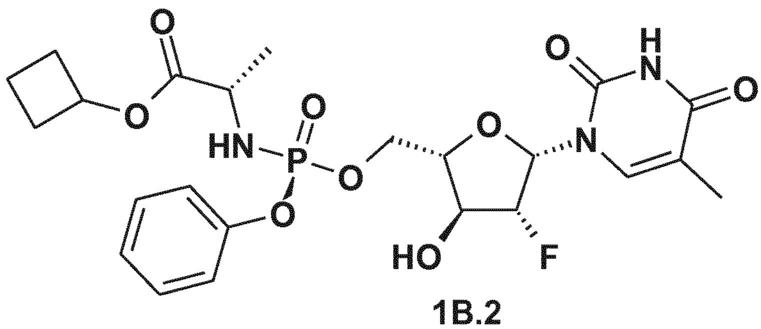

1B.2

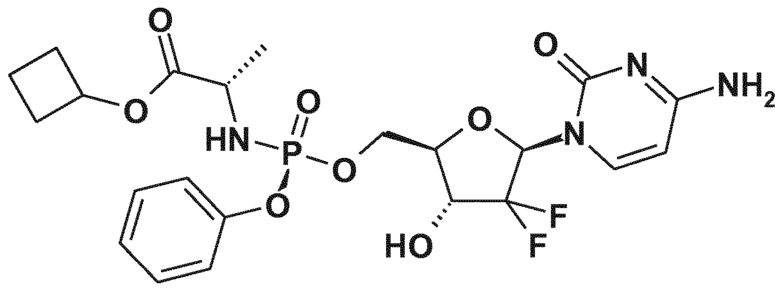

1B.3

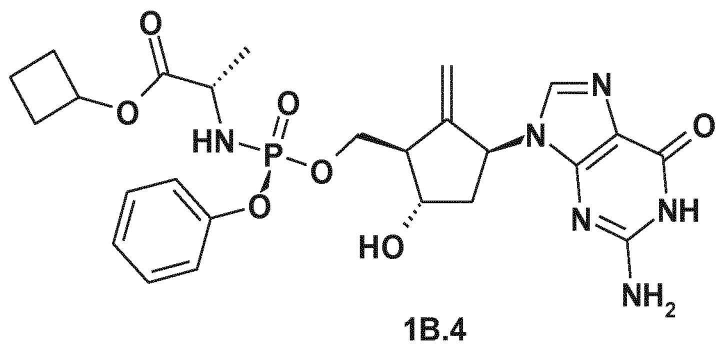

1B.4

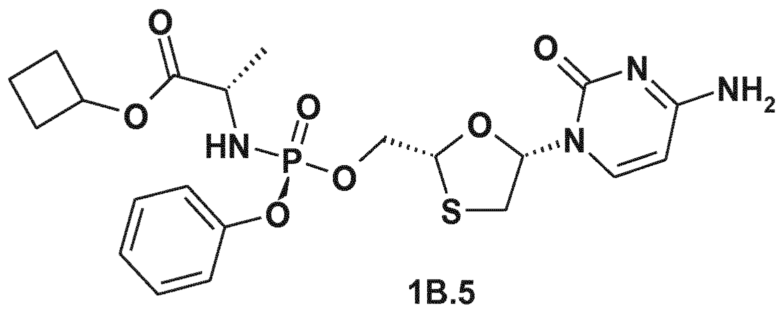

1B.5

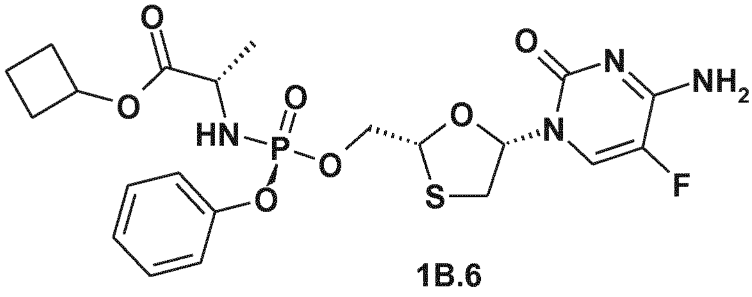

1B.6

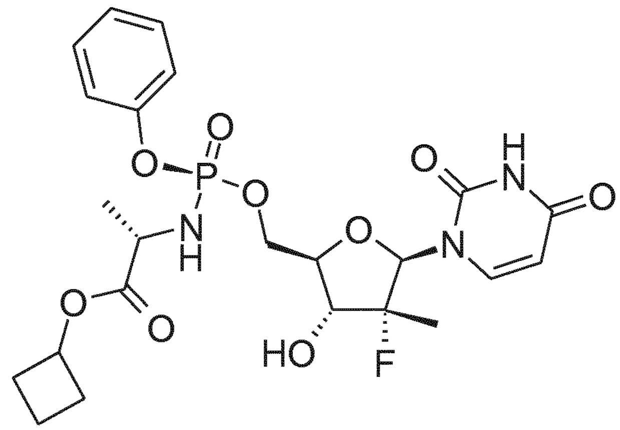

**1B.7**

Surprisingly, the novel prodrugs of formulas 1A.1 and 1A.2 appeared to be more effective than the known TAF prodrugs currently used for the combination treatment of HIV-infected subjects. Indeed, the prodrugs of formulas 1A.1 and 1A.2 metabolize in the peripheral blood mononuclear cells (PBMCs) into TFV and TFV diphosphate leading to increased concentrations and $AUC_{last}$ of metabolites by contrast to those resulted from TDF and TAF metabolism in comparable conditions. Table 1 demonstrates that in the metabolism of the prodrug of formula 1A.1, the values of $C_{max}$ and $AUC_{last}$ of TFV diphosphate (drug) are almost twice as high as the corresponding values observed for the metabolism of its isopropyl analog TAF. Higher $C_{max}$ and $AUC_{last}$ values (Table 1) are also observed for the metabolism of the prodrug of formula 1A.2 as compared to the corresponding values of their isopropyl analog TAF.

**Table 1**. Pharmacokinetic metabolic parameters for the fumarates of formulas 1A.1 and 1A.2 and TAF in PBMC at initial prodrug concentrations of 30 μM

| Prodrug | TFV metabolite formation | | | | TFV diphosphate metabolite formation | | | |
|---|---|---|---|---|---|---|---|---|
| | $C_{max}$, μM | $AUC_{last}$, h·μM | $T_{max}$, h | $T_{1/2}$, h | $C_{max}$, μM | $AUC_{last}$, h·μM | $T_{max}$, h | $T_{1/2}$, h |
| 1A.1 | 0.28 | 13.1 | 4 | 30.7 | 1.76 | 82.6 | 24 | 21.4 |
| 1A.2 | 0.13 | 7.27 | 24 | 64.5 | 1.24 | 63.4 | 24 | 65.1 |
| TAF | 0.11 | 5.86 | 4 | 65.9 | 0.96 | 49.4 | 24 | 73.2 |

The evaluation of antiviral activity for the fumarates of compounds 1A.1 and 1A.2 and TAF in a HIV test using SupT1 cells infected with the NL4.3 HIV strain carrying the GFP-reporter (NL4.3GFP) virus has shown that the fumarate of the compound of formula 1A.1 appears to be the most potent (Table 2) exhibiting both activity and selectivity 1.4 times higher than those of TAF.

**Table 2.** Activity ($EC_{50}$), cytotoxicity ($CC_{50}$), and selectivity index (SI) for the fumarates of formulas 1A.1 and 1A.2 and TAF in an HIV test using SupT1 cells

| Compound | $EC_{50}$, nM | $CC_{50}$, nM | SI = $CC_{50}$ / $EC_{50}$ |
|---|---|---|---|
| 1A.1 fumarate | 27 | >100000 | >3704 |
| 1A.2 fumarate | 38 | >100000 | >2632 |
| TAF | 35 | >100000 | 2857 |

Surprisingly, the novel prodrug of formula 1B.7 and its isotopically enriched analog and crystalline or polycrystalline forms appeared to be more effective HCV NS5B inhibitors than known prodrugs-HCV NS5B inhibitors like Sovaldi® and cyclohexyl ester of formula 2.1.

Indeed, Sovaldi® has against genotype 1b (gT1b) HCV $EC_{50}$=0.045-0.170 μM [http://www.hcvdruginfo.ca/downloads/HCV_Sofosbuvir.pdf] and $EC_{90}$=0.59 μM, while the novel prodrug of formula 1B.7 has $EC_{50}$=15.0-27.0 nM and $EC_{90}$=128.0 nM (Table 3), which means that the novel prodrug of formula 1B.7 more than three times exceeds Sovaldi® in activity.

**Table 3.** Inhibiting activity of the prodrugs of formula 1 B.7, the compounds of formulas 2.1 and 2.2, and Sovaldi® against gT1b HCV NS5B

| Prodrug | 10% FBS | | |
|---|---|---|---|
| | $EC_{50}$, nM | $EC_{90}$, nM | $CC_{90}$, μM |
| 1B.7 | 15.0-27.0 | 128.0 | >100 |
| *Sovaldi® | 45.0-170.0* | 520.0** | >100** |
| 2.1 | | 250.0** | |
| 2.2 | 73.0 | 410.0 | |
| From: *http//www.hcvdruginfo.ca/HCV_Sofosbuvir.pdf;<br>** M. J. Sofia et al. J. Med. Chem. 2010, 53, 7202-7218. | | | |

The half-life of the prodrugs of formula 1B.7 in the S9 fraction of human liver microsome is $T_{1/2}^{hS9}$ = 0.05 h, while Sovaldi® has $T_{1/2}^{hS9}$ = 0.54 h (Table 4), which means that the metabolic rate of the novel prodrug of formula 1B.7 in the S9 fraction of human liver microsome is 11 times faster than that of Sovaldi®.

**Table 4.** Stability and activity of antiviral compositions comprising the novel prodrug of formula 1B.7, the compound of formula 2.1, and Sovaldi®

| ID | $T_{1/2}$ (h) | | | |
|---|---|---|---|---|
| | SGF | SIF | human plasma | human S9 |
| 1B.7 | 12.7 | >24 | >24 | 0.05 |
| Sovaldi® | 22.0* | >24* | >24* | 0.57* |
| 2.1 | 17* | >20* | >24* | 1.4* |
| *from M. J. Sofia et al. J. Med. Chem. 2010, 53, 7202-7218. | | | | |

In addition, the concentration and $AUC_{24h}$ of triphosphate PSI-7409 in the rat liver resulting from the metabolic process of prodrugs 1B.7 are $C_{max}$ = 3 224.0 ng/g and $AUC_{24h}$ = 30 487.0 ng.h/g, respectively, while Sovaldi's similar metabolism leads to $C_{max}$ = 1 934.0 ng/g and $AUC_{24h}$ = 16 796.0 ng h/g (Table 5). This testifies to the fact that the novel prodrug of formula 1B.7 metabolizes into requisite triphosphate PSI-7409 (drug) in the liver almost two times more effectively. Said novel prodrug of formula 1B.7 has even greater efficiency in comparison with known cyclohexyl ester of formula 2.1 (Table 5), which has $EC_{90}$ = 250.0 nM, $T_{1/2}^{hS9}$ = 1.4 h, $C_{max}$ = 557 ng/g and $AUC_{24h}$ = 6 484.0 ng.h/g.

**Table 5**. Pharmacokinetic (PK) parameters of triphosphate PSI-7409 in the rat liver following peroral administration of the prodrugs of formula 1B.7, the compound of formula 2.1, and Sovaldi® for a dose of 50 mg/kg

| PK parameters | Software used for processing PK results | | According to M. J. Sofia et al. J. Med. Chem. 2010, 53, 7202-7218. | |
|---|---|---|---|---|
| | Phoenix™ WinNonlin® 6.3 | GraphPad Prizm | | |
| | Prodrug 1 B.7 | | Sovaldi® | Compound 2.1 |
| $T_{1/2}$, h | 7.2 | 5.5 | | |
| $T_{max}$, h | 8.0 | 4.0 | 4.0 | 2,0 |
| $C_{max}$, ng/g | 3224.0 | 3102.0 | 1934 | 557.0 |
| $C_{24}$ h, ng/g | 320.0 | | | |
| $AUC_{24h}$, ng.h/g | 30487.0 | 30444.0 | 16796.0 | 6487.0 |
| $AUC_{0-inf}$, ng.h/g | 33823.0 | | 18080.0 | 8831.0 |

The results (effect) obtained are surprising, because the prodrug of formula 1B.7 is cyclobutyl ester, which is

not just much more effective than its analog-cyclohexyl ester of formula 2.1, but more active than another analog-cyclopropyl ester of formula 2.2 ($EC_{90}$ = 73.0 nM, $EC_{90}$ = 410.0 nM, see Table 3), which was specially prepared by the inventors to better illustrate said surprising effect.

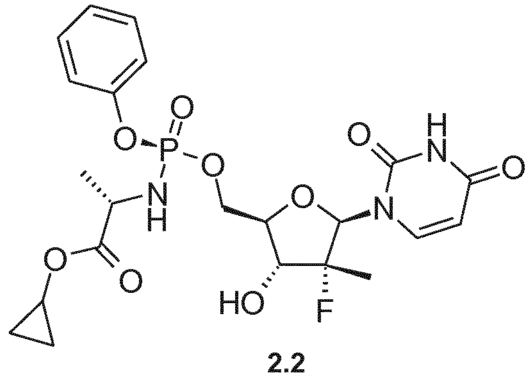

**2.2**

The surprise is that in the series of cycloalkyl esters, the most effective one appeared to be cyclobutyl ester of formula A1.1 with a medium-sized cycloalkyl, while esters with a larger-size cycloalkyl (known cyclohexyl ester of formula 2.1) and a smaller-size cycloalkyl (cyclopropyl ester of formula 2.2 specially prepared by the inventors) appeared to be less effective.

The above data are a convincing proof of the novelty and the level (effectiveness) of this invention.

The subject matter of the present invention is a pharmaceutical composition comprising a prodrug of general formula 1, or its stereoisomer, or their isotopically enriched analog, pharmaceutically acceptable salt, hydrate, solvate, or crystalline or polycrystalline forms, optionally in combination with a pharmaceutically acceptable filler, carrier, additive, and diluent for the treatment of viral infections and/or tumor diseases in mammals.

The prodrugs of general formula 1 may be prepared in a variety of peroral dosage forms and carriers; peroral administration may be effected in the form of tablets, film-coated tablets, hard and soft gelatin capsules, solutions, emulsions, syrups, suspensions. The compounds of this invention are effective when administered in the form of suppositories. Generally, the most convenient route of administration is peroral using a common daily dosage regimen that can be adjusted depending on the severity of disease and a patient's antiviral or antitumor drug reaction.

The prodrug of general formula 1, its stereoisomer, their isotopically enriched analog, pharmaceutically acceptable salt, hydrate, solvate, and crystalline or polycrystalline forms in combination with one or more common excipients, carriers, or diluents may be in the form of pharmaceutical compositions and unit dosage forms thereof. Pharmaceutical compositions and standard dosage forms may consist of ordinary ingredients in usual proportions with or without additional active compounds and dosage forms. The pharmaceutical composition may comprise any appropriate effective amount of active ingredient depending on the prescribed daily dose. Pharmaceutical compositions may be used in the form of solid substances, such as tablets or filled capsules; in the form of semisolid powders, agents with sustained release or liquids such as suspensions, emulsions, or filled capsules for peroral administration; or in the form of suppositories for rectal or vaginal administration. A typical medication will comprise approximately from 5wt% to 95wt% of active compound or the compound. The term "medication" or "dosage form" is meant to include both solid and liquid compositions of active compound, so that it would be clear for a person skilled in the art that the active ingredient may exist in the form of different medications depending on the dose required and pharmacokinetic parameters.

Solid dosage forms include powders, tablets, pills, capsules, suppositories, and dispersible granules. A solid carrier refers to one or more substances that can act as diluents, flavors, solubilizers, lubricants, suspending agents, binding agents, preservatives, disintegrants, or encapsulating material. A powder carrier is generally a fine-grained solid mixed with a fine-grained active component. In the tablets, the active component is usually mixed, in appropriate proportions, with a carrier having a necessary binding capacity and compacted into the desired shape and size. Suitable carriers include, but are not limited to, magnesium carbonate, magnesium stearate, talc, sugar, lactose, pectin, dextrin, starch, gelatin, tragacant, methylcellulose, sodium carboxymethylcellulose, low-melting wax, cocoa butter, and the like. Solid preparations may, in addition to the active ingredient, comprise dyes, flavoring agents, stabilizers, buffers, artificial and natural sweeteners, dispersants, thickeners, solubilizers, and the like.

Liquid compositions are suitable for peroral administration too. Liquid dosage forms include emulsions, syrups, elixirs, and aqueous suspensions. They comprise solid drug forms to be converted to liquid medications immediately before use. Emulsions may be prepared in solutions, for example, in aqueous solutions of propylene glycol, or they may comprise emulsifiers, such as lecithin, sorbitol monooleate, or gum arabic. Aqueous suspensions may be prepared by dispersing a fine-grained active ingredient in water with ductile materials, such as natural or synthetic gums, resins,

methylcellulose, sodium carboxymethylcellulose, and other well-known suspending agents.

The prodrug of general formula 1, its stereoisomer, their isotopically enriched analog, pharmaceutically acceptable salt, hydrate, solvate, and crystalline or polycrystalline forms may be prepared for administration in the form of suppositories. Low-melting wax, such as a mixture of glycerides of fatty acids or cocoa butter, is first melt and then the active ingredient is homogeneously dispersed by, for example, stirring. The molten homogeneous mixture is poured into moulds of a suitable size and allowed to cool and solidify.

The prodrug of general formula 1, its stereoisomer, their isotopically enriched analog, pharmaceutically acceptable salt, hydrate, solvate, and crystalline or polycrystalline form may be prepared for vaginal administration. It will be appropriate to apply suppositories, tampons, creams, gels, pastes, foams, or sprays comprising, in addition to the active ingredient, carriers that are well known in the art.

The subject matter of this invention is the use of the prodrug of general formula 1, its stereoisomer, their isotopically enriched analog, pharmaceutically acceptable salt, hydrate, solvate, and crystalline or polycrystalline forms in the production of a medicament for the treatment of viral and cancer diseases. It is assumed that the prodrug of general formula 1, its stereoisomer, their isotopically enriched analog, pharmaceutically acceptable salt, hydrate, solvate, and crystalline or polycrystalline forms used in the production of dosage forms for the treatment of any antiviral or anticancer disease described herein may be any compound of general formula 1 selected from:

(*S*)-cyclobutyl 2-((*S*)-(((*R*)-1-(6-amino-9*H*-purin-9-yl)propan-2-yloxy)methyl) (phenoxy)phosphorylamino)-propanoate (1A.1),
(*S*)-cyclobutyl 2-((*R*)-(((*R*)-1-(6-amino-9*H*-purin-9-yl)propan-2-yloxy)methyl) (phenoxy)phosphorylamino)-propanoate (1A.2),
(*S*)-cyclobutyl 2-{(*S*)-[(2S,3R,5S)-3-hydroxy-5-(5-methyl-3,4-dihydro-2,4-dioxo-2*H*-pyrimidin-1-yl)-tetrahydrofuran-2-ylmethoxy]-phenoxy-phosphorylamino}-propanoate (1B.1),
(*S*)-cyclobutyl 2-{(*S*)-[(2*S*,3*S*,4*R*,5*S*)-3-hydroxy-4-fluoro-5-(5-methyl-3,4-dihydro-2,4-dioxo-2*H*-pyrimidin-1-yl)-tetrahydrofuran-2-ylmethoxy]-phenoxy-phosphorylamino}-propanoate (1B.2),
(S)-cyclobutyl 2-{(*S*)-[(2*R*,3*R*,5*R*)-5-(4-amino-2-oxo-2*H*-pyrimidin-1-yl)-3-hydroxy-4,4-difluoro-tetrahydrofuran-2-ylmethoxy]-phenoxy-phosphorylamino}-propanoate (1B.3),
(S)-cyclobutyl 2-{(*S*)-[(1*R*,3*S*,5*S*)-3-(2-amino-6-oxo-1,6-dihydro-purin-9-yl)-5-hydroxy-2-methylene-cyclopentylmethoxy]-phenoxy-phosphorylamino}-propanoate (1B.4),
(S)-cyclobutyl 2-{(*S*)-[(2*R*,5*S*)-5-(4-amino-2-oxo-2*H*-pyrimidin-1-yl)-[1,3]oxatiolan-2-ylmethoxy]-phenoxy-phosphorylamino-propanoate (1B.5),
(S)-cyclobutyl 2-{(*S*)-[(2*R*,5*S*)-5-(4-amino-5-fluoro-2-oxo-2*H*-pyrimidin-1-yl)-[1,3]oxatiolan-2-ylmethoxy]-phenoxy-phosphorylamino}-propanoate (1B.6),
(S)-cyclobutyl 2-{(*S*)-[(2*R*,3*R*,4*R*,5*R*)-5-(3,4-dihydro-2,4-dioxo-2*H*-pyrimidin-1-yl)-3-hydroxy-4-methyl-4-fluoro-tetrahydrofuran-2-ylmethoxy]-phenoxy-phosphorylamino}-propanoate (1B.7), their stereoisomers, isotopically enriched analogs, pharmaceutically acceptable salts, hydrates, solvates, or crystalline or polycrystalline forms, either individually or in combination with another compound of this invention. The medicinal agent includes, but is not limited to, any composition claimed herein.

The subject matter of this invention is a method for combination therapy and/or prophylaxis of a subject in need thereof; said method involves administration of a therapeutically effective amount of the prodrug of general formula 1, its stereomer, or their isotopically enriched analog, its pharmaceutically acceptable salt, hydrate, solvate, or crystalline or polycrystalline forms.

The subject matter of this invention is a method for combination therapy and/or prophylaxis of a HIV-infected subject, and said method involves administration of a therapeutically effective amount of the prodrugs of general formulas 1A or 1B or their isotopically enriched analog, pharmaceutically acceptable salt, hydrate, solvate, or crystalline or polycrystalline forms.

The subject matter of this invention is a pharmaceutical composition comprising, as a prodrug, an HCV NS5B polymerase inhibitor of cyclobutyl (S)-2-{(S)-[(2R,3R,4R,5R)-5-(3,4-dihydro-2,4-dioxo-2*H*-pyrimidin-1-yl)-3-hydroxy-4-methyl-4-fluoro-tetrahydrofuran-2-ylmethoxy]-phenoxy-phosphorylamino}-propanoate (1B.7) or an isotopically enriched analog or crystalline or polycrystalline forms thereof.

Preferable is a pharmaceutical composition, which, along with the novel prodrug of formula 1B.7, or its stereomer, isotopically enriched analog, hydrate, solvate, or crystalline or polycrystalline forms, additionally comprises a therapeutically effective amount of a HCV NS5A inhibitor selected from the group including: Daclatasvir (Daklinza, BMS790052) [C. Wang et al. 2012], Hepavivir (AV-4025) [A.V. Ivachtchenko et al. 2014. US 9428491 B2], AV-4067 and AV-4084 [Pat. Appl. US 14/845,333.], AV-4056 and AV-4058 [US 9428491 B2], Ombitasvir (ABT-267) [C. Gardelli et al. Phosphoramidate Prodrugs of 20-C-Methylcytidine for Therapy of Hepatitis C Virus Infection. J. Med. Chem. 2014, 57, 2047-2057 ; WO 2010/144646,], Elbasvir) (MK-8742) [Coburn C. A. et al. ChemMedChem. 2013, 8, 1930-1940 ; WO

2012/040923 ; WO 2012/041014]], or Velpatasvir) (VEL, GS-5816) [WO 2015110048 A1; http://www.accessdata.fda.gov/drugsatfda_docs/nda/2016/208341 Orig1s000PharmR.pdf; http://www.gilead.com/~/media/files/pdfs/medicines/liver-disease/epclusa/epclusa_pi.pdf].

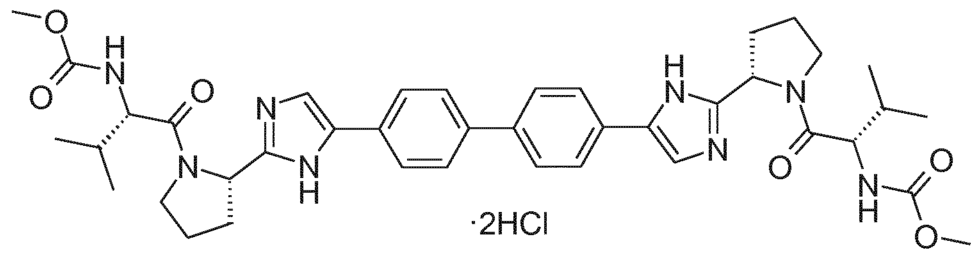

**Daclatasvir (Daklinza, BMS 790052)**

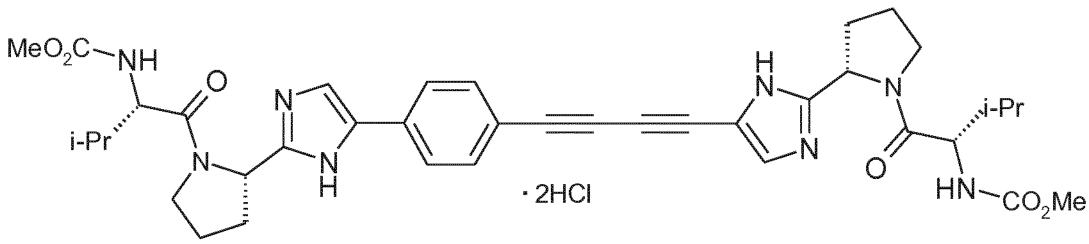

**Hepavivir (AV-4025)**

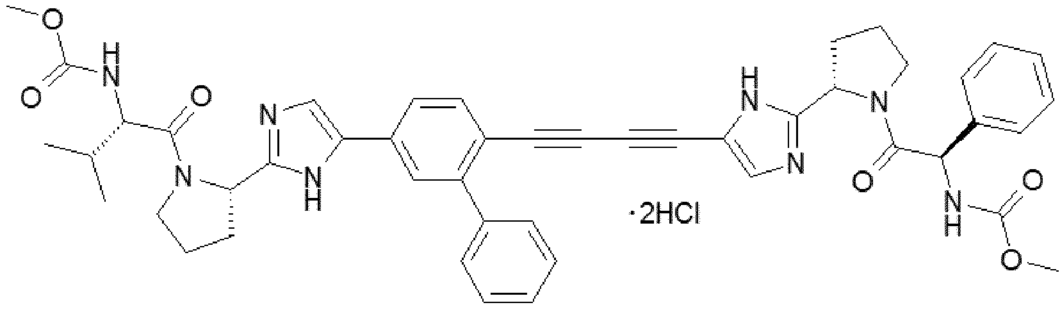

**AV-4067**

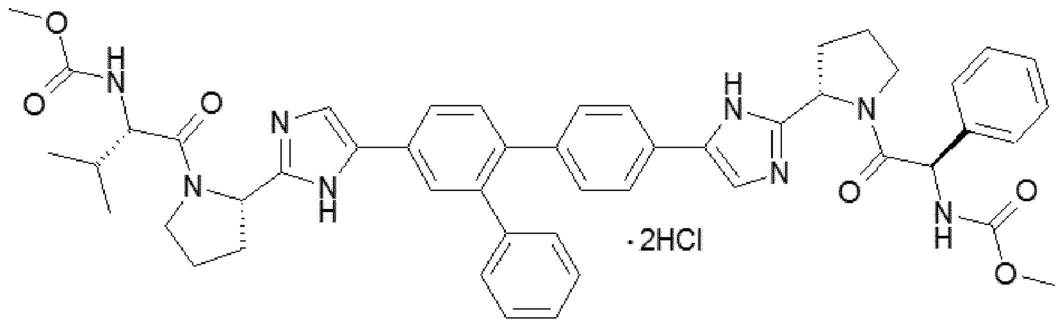

**AV-4084**

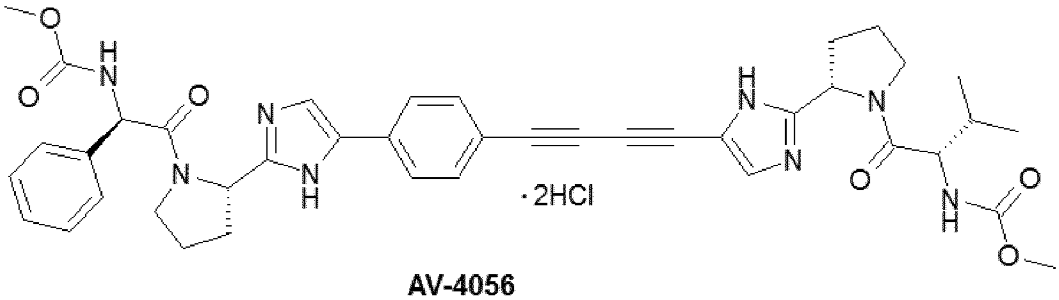

**AV-4056**

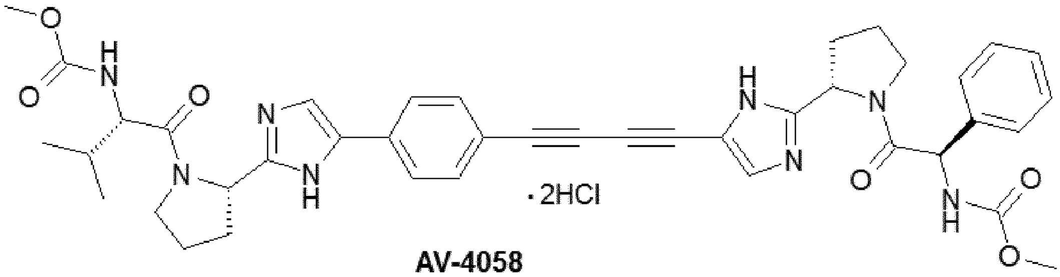

**AV-4058**

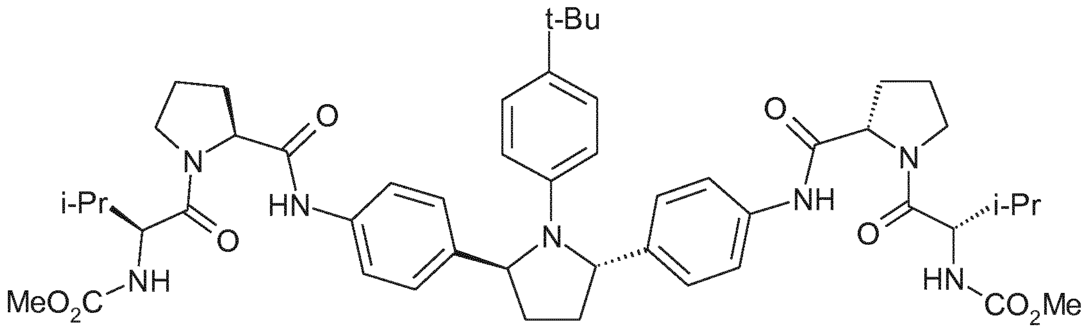

**Ombitasvir (ABT-267)**

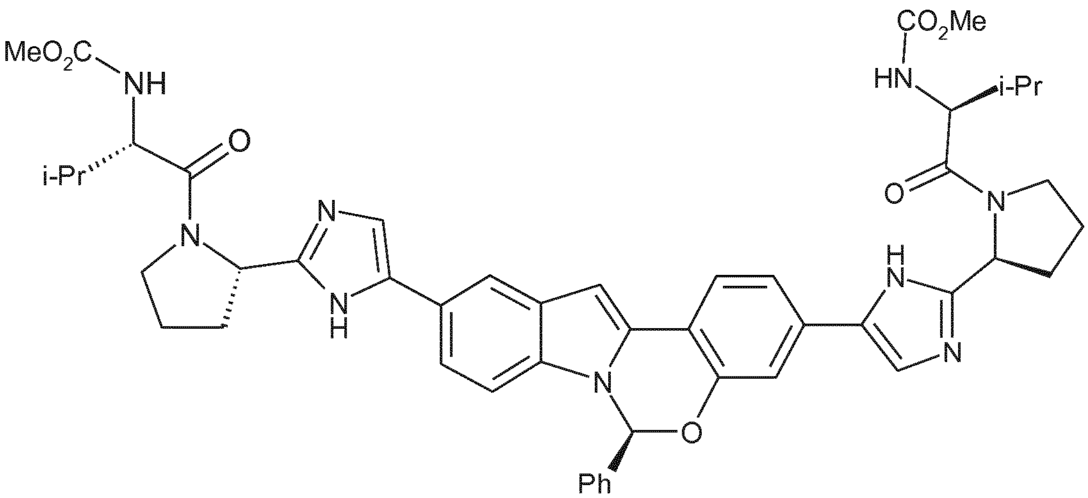

Elbasvir (MK-8742)

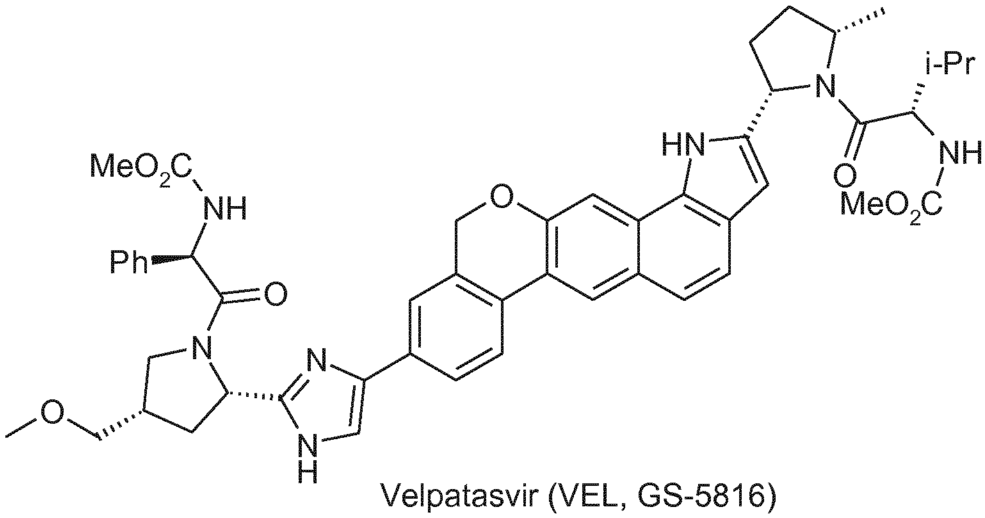

Velpatasvir (VEL, GS-5816)

The subject matter of this invention is a method for combination therapy and/or prophylaxis of a subject in need thereof, and said method includes administration to the subject of an effective amount of the nucleotide of general formula 1 comprising an N-[(S)-1-cyclobutoxycarbonyl]-phosphoramidate moiety, or its stereoisomer, their isotopically enriched analog, pharmaceutically acceptable salt, hydrate, solvate, or crystalline or polycrystalline forms of the pharmaceutical composition of this invention, which may additionally comprise a therapeutically effective amount of one or more antiviral or anticancer agents, wherein agents are administered simultaneously or alternatively. It is understood that there may be any time span between the successive administrations of agents.

A further subject matter of this invention is a method for combination therapy and prophylaxis of a HIV-infected subject, said method involving administration to the subject of an effective amount of the prodrug of formula 1B.1, its stereoisomer, isotopically enriched analog, pharmaceutically acceptable salt, hydrate, solvate, or crystalline or polycrystalline forms or the pharmaceutical composition of this invention and a therapeutically effective amount of one or more anti-HIV agents, wherein agents are administered simultaneously or alternatively. It is understood that there may be any time span between the successive administrations of agents.

Yet another subject matter of this invention is a method for the treatment of a HCV-infected subject in need thereof, and said method includes administration to the subject of an effective amount of the prodrug of formula 1B.7, its stereoisomer, isotopically enriched analog, pharmaceutically acceptable salt, hydrate, solvate, or crystalline or polycrystalline forms or the pharmaceutical composition of this invention and a therapeutically effective amount of another antiviral agent-an HCV NS5A inhibitor, wherein the agents are administered simultaneously or alternatively. It is understood that there may be any time span between the successive administrations of agents.

When the prodrug of general formula 1, its stereoisomer, isotopically enriched analog, pharmaceutically acceptable salt, hydrate, solvate, or crystalline or polycrystalline forms are administered in combination with another antiviral or anticancer agent, the activity may be increased against the initial activity of the prodrug. In combination therapy, the administration of agents may be simultaneous or successive regarding the prodrug of general formula 1. The notion "simultaneous administration" as used herein thus means administration of agents at the same time or at different times. The administration of two or more agents at the same time may be performed by using one preparative form comprising two or more active ingredients or, in essence, by simultaneously administering two or more dosage forms with one active ingredient. It is to be understood that any reference to therapy as used herein covers prophylaxis as well. In addition, the term ""therapy" of viral infection, as used herein, includes treatment or prophylaxis of a disease or a condition associated with a mediated viral infection, or clinical symptoms thereof.

**Best embodiment**

The present invention will now be described in terms of certain embodiments which are not intended to limit its scope. On the contrary, the present invention covers all alternatives, modifications, and equivalents that can be included within the scope of the claims. Thus, the following examples, which include specific embodiments, will illustrate this invention without limiting it.

**Example 1**. General synthetic protocol for the prodrugs of general formula 1A (Scheme 1).

Scheme 1

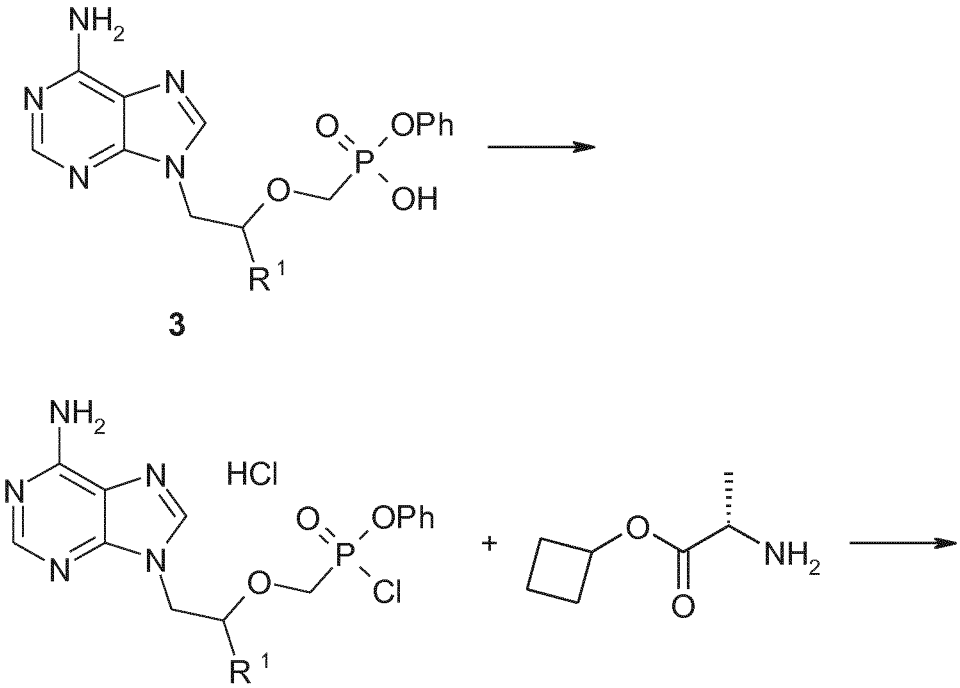

3

1A

4 5

where $R^1$ is as indicated above.

Thionyl chloride (3 ml, 40 mmol) was added dropwise with stirring to a suspension of [(R)-2-(6-amino-purin-9-yl)-1-methyl-ethoxymethyl]phosphonic acid monophenyl ether (3.63 g, 10 mmol) (3) [WO 2013116720] in sulfolane (14 ml) and dichloromethane (12 ml). The mixture was refluxed at 50-55°C under low Ar flow for 15 h. Then, to remove volatile components, vacuum (via a membrane pump) was applied to the flask for 2 h at 50-55°C. The reaction mixture was allowed to cool down to 30°C, and a mixture of dichloromethane (10 ml) and dry acetonitrile (40 ml) was added with stirring. The reaction mixture containing chloride (4) was cooled down to (-60)-(-50)°C, and a solution of L-alanine cyclobutyl ester (2) (1.546 g, 12 mmol) and triethylamine (4.172 ml, 30 mmol) in 6 ml of acetonitrile was added. The mixture was allowed to heat slowly to room temperature, diluted with dichloromethane (100 ml) and spread onto about 100 ml of silica gel on a glass filter. The product was extracted by dry flash chromatography eluting first with dichloromethane, then with a 30% solution of acetone in dichloromethane, and finally with pure tetrahydrofuran to afford 2 g of (*S*)-cyclobutyl 2-(((*R*)-1-(6-amino-9*H*-purin-9-yl)propan-2-yloxy)methyl)(phenoxy)phosphorylamino)-propanoate (1A) consisting of a mixture of phosphorus stereoisomers of formulas 1A.1 and 1A.2. The stereoisomers of formulas 1A.1 and 1A.2 were separated by HPLC on a Phenomenex Amylose-2 AXIA-Pac 250×21.20 mm optical column in an isocratic system of AcCN:EtOH:HCOOH 200:20:0.5 (flow rate 20 ml/min) with a 254-nm UV detector. The resulting stereoisomers of formulas 1A.1 and 1A.2 were recrystallized from 100 ml of acetonitrile with an equimolar amount of fumaric acid to afford an $S_P$-isomer: (S)-cyclobutyl 2-((*S*)-(((*R*)-1-(6-amino-9*H*-purin-9-yl)propan-2-yloxy)methyl)(phenoxy) phosphorylamino)propanoate fumarate (1A.1), LC-MS (ESI) 489 $(M+H)^+$. $^1$H NMR (DMSO-$d_6$, 300 MHz) $\delta$ 8.14 (s, 1H), 8.10 (s, 1H), 7.30 (m, 2H), 7.19 (s, 2H), 7.14 (m, 1H), 7.06 (m, 2H), 6.63 (s, 2H), 5.64 (t, $J$ = 11.1 Hz, 1H), 4.86 (p, $J$ = 7.2 Hz, 1H), 4.27 (dd, $J_1$ = 14.4 Hz, $J_2$ = 3.0 Hz, 1H), 4.14 (dd, $J_1$ = 14.4 Hz, $J_2$ = 6.6 Hz, 1H), 3.85 (m, 4H), 2.23 (m, 2H), 1.94 (m, 2H), 1.72 (m, 1H), 1.59 (m, 1H), 1.13 (d, $J$ = 6.9 Hz, 3H), 1.07 (d, $J$ = 6.6 Hz, 3H). $^{31}$P NMR (DMSO-$d_6$, 121.5 MHz) $\delta$ 22.05 and an $R_P$-isomer: (*S*)-cyclobutyl 2-((*R*)-(((*R*)-1-(6-amino-9*H*-purin-9-yl)propan-2-yloxy)methyl)(phenoxy)phosphorylamino)propanoate fumarate (1A.2), LC-MS (ESI) 489 $(M+H)^+$. $^1$H NMR (DMSO-$d_6$, 300 MHz) $\delta$ 8.14 (s, 1H), 8.12 (s, 1H), 7.34 (m, 2H), 7.21 (s, 2H), 7.15 (m, 1H), 7.11 (m, 2H), 6.63 (s, 2H), 5.53 (dd, $J_1$ = 12.0 Hz, $J_2$ = 10.5 Hz, 1H), 4.82 (p, $J$ = 7.5 Hz, 1H), 4.29 (dd, $J_1$ = 14.4 Hz, $J_2$ = 3.6 Hz, 1H), 4.20 (dd, $J_1$ = 14.4 Hz, $J_2$ = 5.7 Hz, 1H), 3.98 (m, 1H), 3.86 (m, 3H), 2.21 (m, 2H), 1.91 (m, 2H), 1.69 (m, 1H), 1.57 (m, 1H), 1.13 (d, $J$ = 6.9 Hz, 3H), 1.05 (d, $J$ = 6.3 Hz, 3H). $^{31}$P NMR (DMSO-$d_6$, 121.5 MHz) $\delta$ 22.86.

**Example 2**. Synthetic protocol for (*S*)-cyclobutyl 2-(pentafluorophenoxy-phenoxy-phosphorylamino)-propanoates (7, 7.1) (Scheme 2).

Scheme 2

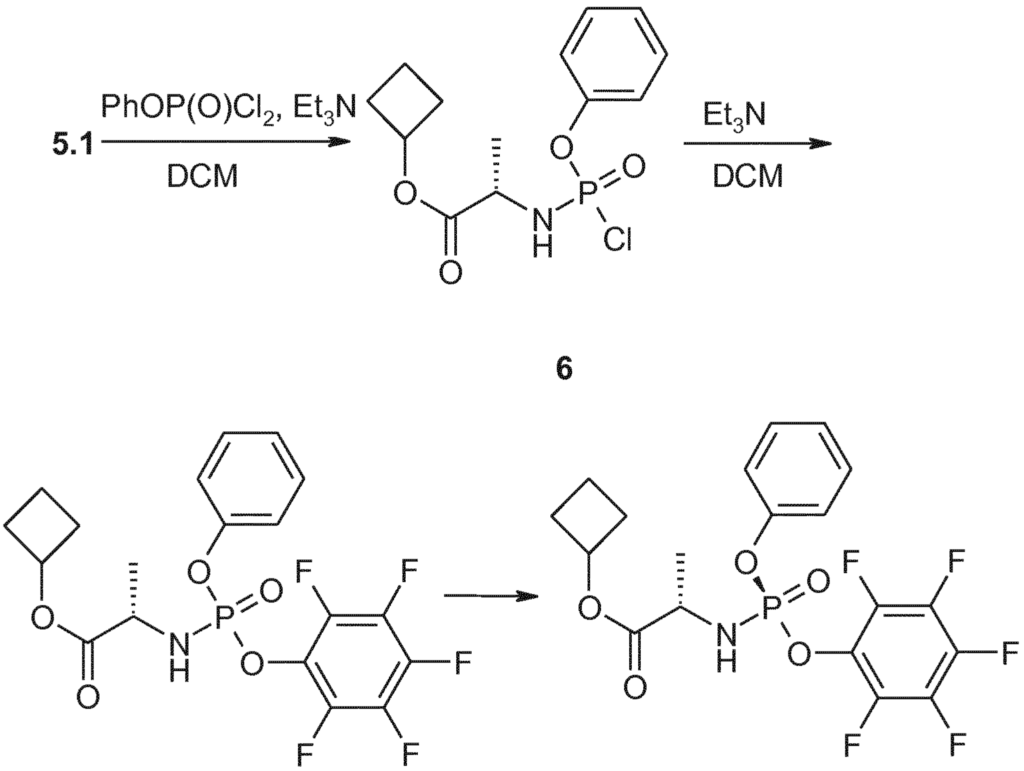


6

7

7.1

Phenyl dichlorophosphate (16.9 g, 80.2 mmol, 1 eq.) was added to a solution of cyclobutyl L-alanine hydrochloride (14.4 g, 80.2 mmol, 1 eq.) (5.1) [WO 2014033617 A1] in DCM (214 ml). The mixture was cooled to (-75)-(-70) °C, and at that temperature a solution of triethylamine (16.2 g, 160.4 mmol, 2 eq.) in dichloromethane (16 ml) was added. The mixture was stirred 30 min at -70°C and then heated to -20 °C. To the reaction mixture containing chloride 6, a solution of pentafluorophenol (14.6 g, 79.4 mmol, 0.99 eq.) in 105 ml of dichloromethane was added at (-20)-(-10)°C, then, a solution of triethylamine (8.1 g, 80.2 mmol, 1 eq.) in 8 ml of dichloromethane was added at (-20)-(-10)°C, and the mixture was stirred overnight at room temperature. The mixture was evaporated in vacuum until dry, and then ethyl acetate (500 ml) and water (500 ml) were added. The organic layer was separated and washed consecutively with 200 ml water, a 5% $NaHCO_3$ aqueous solution, and a saturated salt solution, then dried over dried over $Na_2SO_4$ and evaporated in vacuum until dry. To the residue, which was (*S*)-cyclobutyl 2-(pentafluorophenoxy-phenoxy-phosphorylamino)-propanoate of formula 7, a mixture (200 ml) of hexane-ethyl acetate (6:1) was added, and the mixture was stirred overnight at room temperature, then filtered, washed with 50 ml of a hexane-ethyl acetate (6:1) mixture, and air dried to afford 16.7 g of a product, which was recrystallized from 500 ml of a hexane and ethyl acetate mixture (4:1) to afford 13.8 g of (*S*)-cyclobutyl 2-((*R*)-(pentafluorophenoxy-phenoxy-phosphorylamino)-propanoate (7.1). $^1H$ NMR (400 MHz, $CDCl_3$) δ 7.42-7.32(m, 2H), 7.28-7.19(m, 3H), 5.02(p, J=7.6Hz, 1H), 4.23-4.1(m, 1H), 4.01-3.88(m, 1H), 2.42-2.3(m, 2H), 2.14-1.99(m, 2H), 1.89-1.77(m, 1H), 1.72-1.59(m, 1H), 1.48(d, J=7.2Hz, 3H).

Similarly, but starting from L-alanine hydrochloride (8), (*S*)-cyclopropyl 2-(pentafluorophenoxy-phenoxy-phosphorylamino)-propanoate 9 and (*S*)-cyclopropyl 2-((*R*)-(pentafluorophenoxy-phenoxy-phosphorylamino)-propanoate (9.1) were obtained. $^1H$ NMR (400 MHz, $CDCl_3$) δ 0.71-0.77 (m, 4H), 1.45 (d, J=6.8 Hz, 3H), 3.98 (m, 1H), 4.17 (m, 1H), 7.25 (m, 3H), 7.36 (m, 2H).

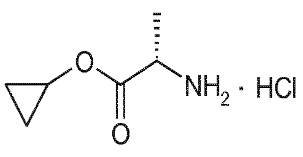

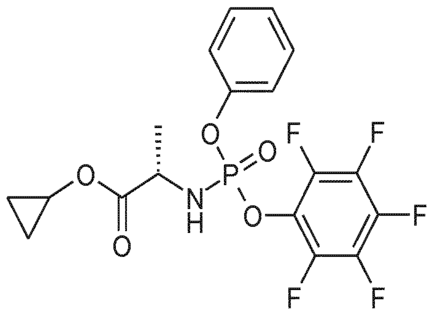

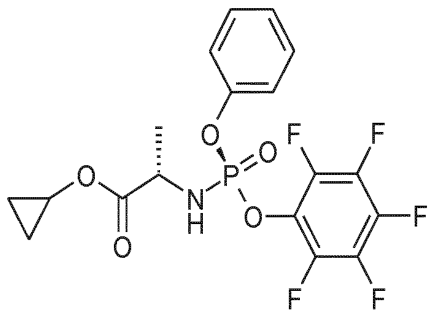

8 9 9.1

**Example 3.** General synthetic protocol for the prodrugs of general formula 1 B (Scheme 3).

Scheme 3

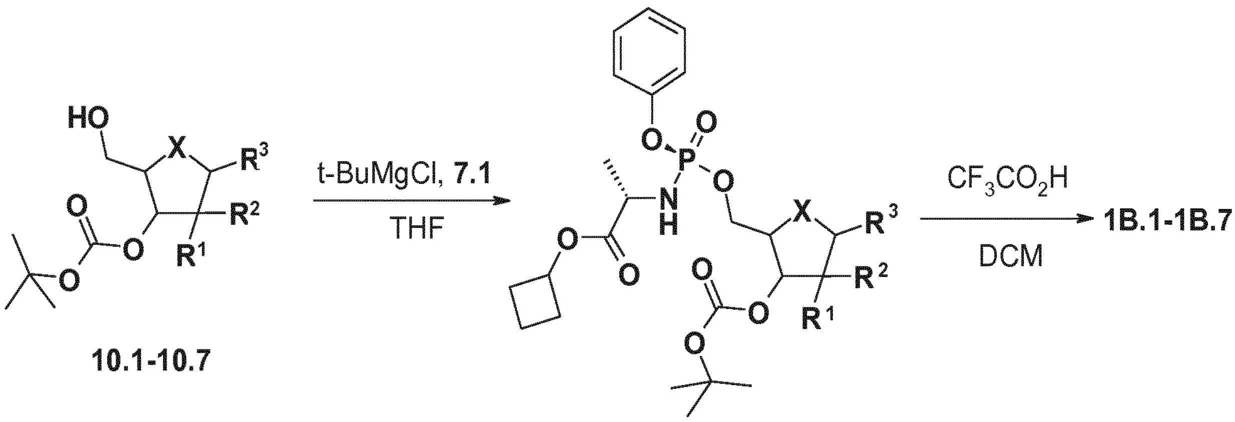


10.1-10.7

11.1-11.7

1B.1-1B.7

To a solution of *tert*-butyl (2*R*,3*R*,4*R*,5*R*)-5-(2,4-dioxo-3,4-dihydropyrimidin-1 (2*H*)-yl)-2-(hydroxymethyl)-4-methyl-4-fluorotetrahydrofuran-3-ylcarbonate (5 g, 13.9 mmol, 1 eq.) (10.7) in 165 ml of tetrahydrofuran, a 1M solution (31.3 ml, 31.3 mmol, 2.25 eq.) of *tert*-butylmagnezium chloride was added under argon, and the resulting mixture was stirred 30 min. at room temperature. Then, a solution of (*S*)-cyclobutyl 2-((R)-(pentafluorophenoxy-phenoxy-phosphorylamino)-propanoate (7.8 g, 16.7 mmol, 1.2 eq.) (7.1) in 30 ml of tetrahydrofuran was added with stirring at 0-5°C. The reaction mixture was stirred 24 h at room temperature under argon, then methanol (10 ml) was gradually added, and the mixture was concentrated in vacuum. The residue was dissolved in 500 ml of ethyl acetate, washed with a 5% citric acid, a $NaHCO_3$ solution, and a saturated salt solution, then dried over $Na_2SO_4$ and evaporated on a rotary evaporator until dry to afford 12.7 g of (S)-cyclobutyl 2-((R)-(((2*R*,3*R*,4*R*,5*R*)-3-(*tert*-butoxycarbonyloxy)-5-(2,4-dioxo-3,4-dihydropyrimidin-1(2*H*)-yl)-4-methyl-4-fluorotetrahydrofuran-2-yl)methoxy)(phenoxy)phosphorylamino)propanoate (11.7) appearing as a yellow oil. $^1H$ NMR (400 MHz, CDCl3) $\delta$ 8.77(s, 1H), 7.52(d, J=8Hz, 1H), 7.39-7.29(m, 2H), 7.25-7.13(m, 3H), 6.19(d, J=18.4Hz, 1H), 5.52(d, J=8Hz, 1H), 5.08-4.93(m, 2H), 4.63-4.53(m, 1H), 4.4-4.25(m, 2H), 4.08-3.91(m, 2H), 2.42-2.28(m, 2H), 2.13-1.97(m, 2H), 1.88-1.75(m, 1H), 1.71-1.58(m, 1H), 1.52(s, 9H), 1.46-1.36(m, 6H). To a solution of (*S*)-cyclobutyl 2-((*R*)-(((2*R*,3*R*,4*R*,5*R*)-3-(*tert*-butoxycarbonyloxy)-5-(2,4-dioxo-3,4-dihydropyrimidin-1(2*H*)-yl)-4-methyl-4-fluorotetrahydrofuran-2-yl)methoxy) (phenoxy)phosphorylamino) propanoate (8.9 g, 13.8 mmol, 1 eq.) (11.7) in 120 ml of dichloromethane, trifluoroacetic acid (120 ml) was added at (-10)-(0)°C. The mixture was stirred overnight at room temperature and then dried on a rotary evaporator. The residue was dissolved in 500 ml of dichloromethane and diluted with a 5% aqueous solution of $Na_2CO_3$ until pH was approximately 8. The organic layer was separated, dried over $Na_2SO_4$, filtered, and dried on a rotary evaporator in vacuum. The product was purified using column chromatography (silica gel, ethyl acetate:hexane = 1: 1-2: 11: 0). The product was repurified using column chromatography (silica gel, dichloromethane : methanol = 19: 1-9: 1) to afford 5.4 g of (*S*)-cyclobutyl 2-((*S*)-(((2R,3R,4R,5R)-5-(2,4-dioxo-3,4-dihydropyrimidin-1(2*H*)-yl)-3-hydroxy-4-methyl-4-fluorotetrahydrofuran-2-yl)methoxy)(phenoxy) phosphorylamino)propanoate (1B.7). LC-MS (ESI) 542 (M+H)$^+$. $^1$H NMR (400 MHz, DMSO-$d_6$) δ 11.51 (brs, 1H), 7.56 (d, $J$ = 8.0 Hz, 1H), 7.38 (m, 2H), 7.23 (m, 2H), 7.19 (m, 1H), 6.03 (m, 2H), 5.84 (d, $J$ = 6.8 Hz, 1H), 5.55 (dd, $J_1$ = 8.0 Hz, $J_2$ = 1.2 Hz, 1H), 4.85 (p, $J$ = 7.2 Hz, 1H), 4.37 (m, 1H), 4.27 (m, 1H), 4.01 (m, 1H), 3.83 (m, 2H), 2.23 (m, 2H), 1.95 (m, 2H), 1.71 (m, 1H), 1.56 (m, 1H), 1.25 (d, $J$ = 22.8 Hz, 3H), 1.23 (d, $J$ = 6.8 Hz, 3H).

Recrystallization of the prodrug of formula 1B.7 from various solvents leads to polycrystalline or crystalline forms. Thus, recrystallization from a mixture of ethyl acetate with methyl-*tert*-butyl ether (1:1), ethanol, ethyl acetate, and a mixture of acetic acid with water affords the prodrug of formula 1B.7 in polycrystalline forms generally comprising an orthorhombic phase with unit cell parameters of *a* = 28.1056(8) A, *b* = 16.8998(4) A, *c* = 5.25380(12) A and a monoclinic phase with unit cell parameters of *a* = 16.2770(6) A, *b* = 16.9117(8) A, *c* = 5.20429(15) A, β = 117.822(2)°.

Recrystallization of the prodrug of formula 1B.7 from a mixture of dimethylwith water leands to a white crystalline substance consisting of an orthorhombic phase with unit cell parameters of ***a*** = 28.1056(8) A, ***b*** = 16.8998(4) A, ***c*** = 5.25380(12) A.

Crystalline and polycrystalline forms have similar solubility values after recrystallization from various solvents at pH = 2 and pH = 7 varying from 0.18 to 0.25 mg/ml. The exception is a polycrystalline sample obtained from recrystallization from dimethyl sulfoxide, the solubility of which is a little higher and varies from 0.63 to 0.67 mg/ml (Table 6).

**Table 6**. Kinetic solubility of polycrystalline and crystalline forms of prodrugs of formula 1B.7 при длине волны 260 nm

| Prodrug 1B.7 recrystallized from | Form of prodrug 1B.7 | Solubility, mg/ml | | | |
|---|---|---|---|---|---|
| | | at pH = 2 | | at pH = 7 | |
| | | value | SD | value | SD |
| Mixture of ethyl acetate with methyl-*tert*-butyl ester | polycrystal | 0.25 | 0.00 | 0.24 | 0.001 |
| Ethanol | polycrystal | 0.20 | 0.00 | 0.20 | 0.003 |
| Ethyl acetate | polycrystal | 0.22 | 0.00 | 0.22 | 0.002 |
| Acetic acid | polycrystal | 0.63 | 0.009 | 0.67 | 0.041 |
| Dimethyl sulfoxide | crystal | 0.18 | 0.00 | 0.18 | 0.003 |

Similarly to 1B.7, compounds (*S*)-cyclobutyl 2-{(*S*)-[(2S,3R,5S)-3-hydroxy-5-(5-methyl-3,4-dihydro-2,4-dioxo-2*H*-pyrimidin-1-yl)-tetrahydrofuran-2-ylmethoxy]-phenoxy-phosphorylamino}-propanoate (1B.1), LC-MS (ESI) 524 (M+H)$^+$; (*S*)-cyclobutyl 2-{(*S*)-[(2*S*,3*S*,4*R*,5*S*)-3-hydroxy-4-fluoro-5-(5-methyl-3,4-dihydro-2,4-dioxo-2*H*-pyrimidin-1-yl)-tetrahydrofuran-2-ylmethoxy]-phenoxy-phosphorylamino}-propanoate (1B.2), LC-MS (ESI) 542 (M+H)$^+$; (*S*)-cyclobutyl 2-{(*S*)-[(2*R*,3*R*,5*R*)-5-(4-amino-2-oxo-2*H*-pyrimidin-1-yl)-3-hydroxy-4,4-difluoro-tetrahydrofuran-2-ylmethoxy]-phenoxy-phosphorylamino}-propanoate (1B.3), LC-MS (ESI) 545 (M+H)$^+$ and (*S*)-cyclobutyl 2-{(S)-[(1*R*,3*S*,5*S*)-3-(2-amino-6-oxo-1,6-dihydro-purin-9-yl)-5-hydroxy-2-methylene-cyclopentylmethoxy]-phenoxy-phosphorylamino}-propanoate (1B.4), (*S*)-cyclobutyl 2-{(*S*)-[(2*R*,5*S*)-5-(4-amino-2-oxo-2*H*-pyrimidin-1-yl)-[1,3]oxatiolan-2-ylmethoxy]-phenoxy-phosphorylamino-propanoate (1B.5), LC-MS (ESI) 511 (M+H)$^+$ and (*S*)-cyclobutyl 2-{(*S*)-[(2*R*,5S)-5-(4-amino-5-fluoro-2-oxo-2*H*-pyrimidin-1-yl)-[1,3]oxatiolan-2-ylmethoxy]-phenoxy-phosphorylamino}-propanoate (1B.6), LC-MS (ESI) 529 (M+H)$^+$ were prepared.

**Example 4.** Synthetic protocol for the (S)-cyclopropyl 2-((*S*)-(((2*R*,3*R*,4*R*,5*R*)-5-(2,4-dioxo-3,4-dihydropyrimidin-1(2*H*)-yl)-3-hydroxy-4-methyl-4-fluoro-tetrahydrofuran-2-yl)methoxy)-(phenoxy)-phosphorylamino)-propanoate (2.2) prodrug (Scheme 4).

Scheme 4

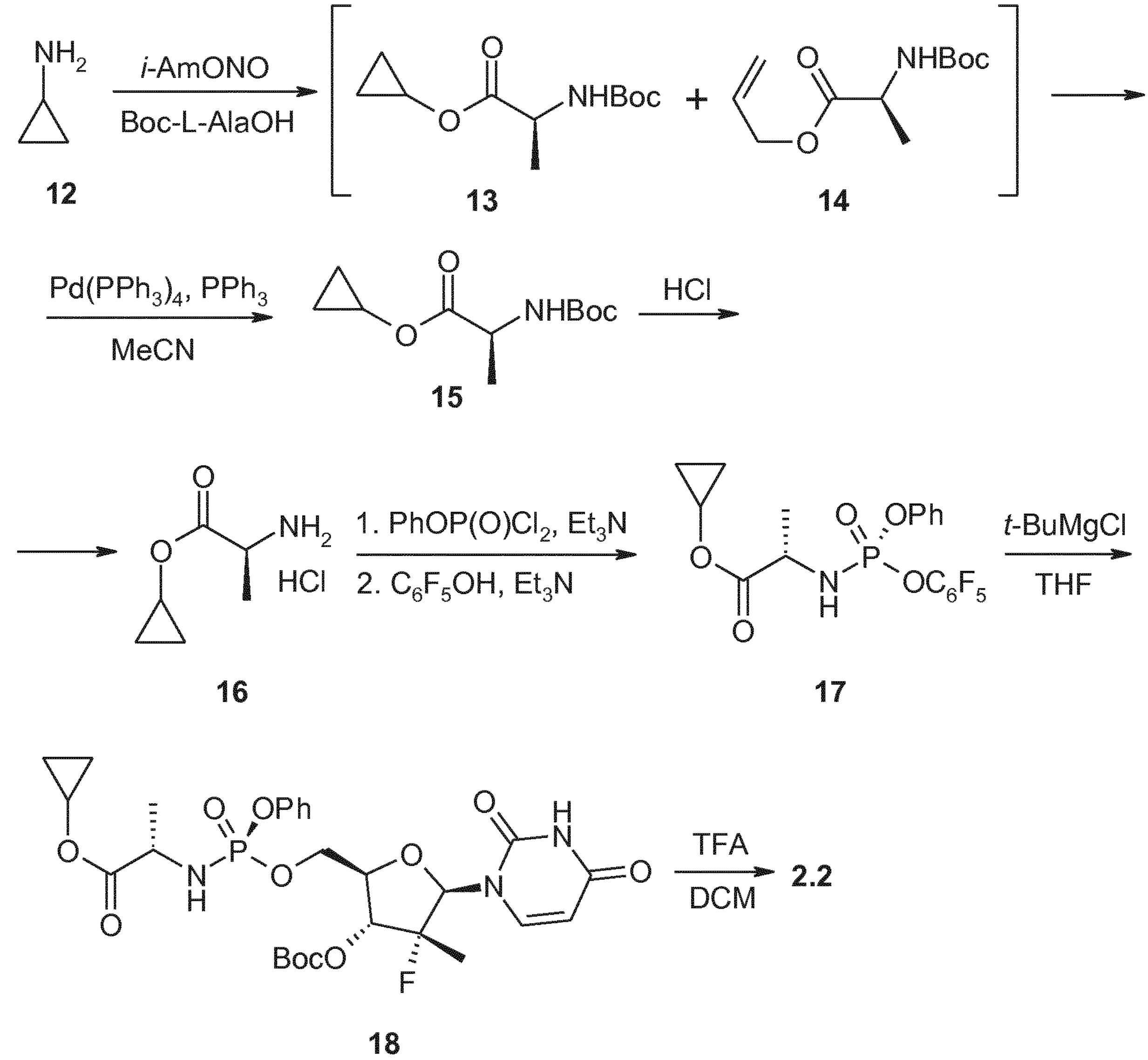


Cyclopropylamine (12: 4.06 ml, 58.8 mmol) and Boc-L-alanine (22.2 g, 58.8 mmol) were dissolved in 250 ml of chloroform, and isoamyl nitrite (7.9 ml, 58.8 mmol) was added under cooling with ice. The mixture was stirred under cooling for 16 h, evaporated till dry, and chromatographed on silica gel (eluting with ethyl acetate : hexane 1:8) to afford 7.68 g (57%) of a mixture of cyclopropyl ester of formula 13 and allyl ether of formula 14 in a ratio of 1:4 (based on $^1$H NMR). The resulting mixture of ester 13 and ether 14 was dissolved in 120 ml of acetonitrile, whereupon triphenylphosphene (446 mg, 1.7 mmol) and $Pd(PPh_3)_4$ (984 mg, 0.85 mmol) were added under argon. The solution was cooled with ice and diluted with a solution of pyrrolidine (2.49 g, 35 mmol) in acetonitrile (30 ml). The mixture was stirred 16 h under argon at 0-4 °C, evaporated till dry, and chromatographed on silica gel (eluting with ethyl acetate : hexane 1:8) to afford 1.38 g of (*S*)-cyclopropyl 2-(*tert*-butoxycarbonylamino)propanoate (15). $^1$H NMR ($CDCl_3$, 400 MHz) δ 5.04 (br.s., 1H), 4.26 (m, 1H), 4.19 (m, 1H), 1.46 (c, 9H), 1.37 (d, *J* = 7.2 Hz, 3H), 0.74 (m, 4H).

To a solution of the compound of formula 15 (3.5 g, 15.3 mmol) in 10 ml of dioxane, 20 ml of a 3M HCl solution in dioxane was added. The mixture was stirred 2 h at room temperature and evaporated till dry to afford 2.53 g of (*S*)-cyclopropyl 2-amino-propanoate hydrochloride (16). $^1$H NMR (DMSO-$d_6$, 300 MHz) δ 8,65 (br.s., 3H), 4,19 (m, 1H), 3,99 (q, *J* = 6.9 Hz, 1H), 1.39 (d, *J* = 6.9 Hz, 3H), 0.73 (m, 4H).

To a solution of the compound of formula 16 (2.53 g, 15.3 mmol) and phenyldichlorophosphate (3.23 g, 15.3 mmol) in 50 ml of dichloromethane cooled down to -70°C, a solution of triethylamine (4.26 ml, 30.6 mmol) in 10 ml of dichloromethane was added dropwise. The temperature of the reaction mixture was then allowed to rise to -10°C, and a mixture of pentafluorophenol (2.82 g, 15.3 mmol) and triethylamine (2.13 ml, 15.3 mmol) in 15 ml of dichloromethane,

which had been prepared beforehand, was added dropwise. Upon completion of addition, the reaction mixture was stirred for 12 h at room temperature, then evaporated, and the residue was treated with 50 ml of benzene. The residue was filtered off and washed with 15 ml of benzene. The filtrate was washed with a saturated sodium hydrocarbonate solution, dried over sodium sulfate, and evaporated. A mixture of ethyl acetate:hexane 1:4 at a rate of 8 ml per 1 g of the substance was added to the residue, and the resulting mixture was vigorously stirred for 16 h. The residue was filtered off and recrystallized from a mixture of ethyl acetate : hexane 1:4 to afford 1.02 g (14%) of (S)-cyclopropyl 2-((S)-(perfluorophenoxy)-(phenoxy)-phosphorylamino)-propanoate (17). LC-MS (ESI) 452 $(M+H)^+$. $^1H$ NMR ($CDCl_3$, 300 MHz) $\delta$ 7.38 (m, 2H), 7.26 (m, 3H), 4.7 (m, 1H), 3.96 (m, 1H), 1.46 (d, *J* = 7.2 Hz, 3H), 0.74 (m, 4H).

To a solution of tert-butyl (2*R*,3*R*,4*R*,5*R*)-5-(2,4-dioxo-3,4-dihydropyrimidin-1(2*H*)-yl)-2-(hydroxymethyl)-4-methyl-4-fluoro-tetrahydrofuran-3-yl carbonate (820 mg, 1.85 mmol) in 25 ml of dry THF cooled with ice, a 1M solution of *t*-BuMgCl in THF (4 ml, 0.4 mmol) was added dropwise. The cooling was terminated and the reaction mixture was stirred 0.5 h at room temperature, then cooled with ice again, and a solution of the compound of formula 17 (1.02 g, 2.18 mmol) in THF was added dropwise. The reaction mixture was stirred for 12 h at room temperature and then treated with a saturated ammonium chloride solution. The organic phase was separated, and the aqueous phase was extracted with dichloromethane. The combined extracts were dried over sodium sulfate and filtered to afford ~1.16 g of (S)-cyclopropyl 2-((*S*)-(((2*R*,3*R*,4*R*,5*R*)-3-(*tert*-butoxycarbonyloxy)-5-(2,4-dioxo-3,4-dihydropyrimidin-1(2*H*)-yl)-4-methyl-4-fluoro-tetrahydrofuran-2-yl)methoxy)-(phenoxy)-phosphorylamino)-propanoate (18), which was used at the next stage as such. LC-MS (ESI) 628 $(M+H)^+$.

To a solution of the compound of formula 18 (~1.16 g, 1.85 mmol) in 20 ml of dichloromethane, trifluoroacetic acid (20 ml) was added under cooling with ice. The reaction mixture was stirred for 3 h and then concentrated *in vacuo.* The residue was dissolved in dichloromethane, diluted with water, and neutralized with sodium hydrocarbonate. The organic layer was separated, dried over sodium sulfate, and evaporated till dry. The residue was chromatographed on silica gel (chloroform:methanol) and recrystallized from a mixture of ethyl acetate : MTBE to afford 505 mg of (*S*)-cyclopropyl 2-((*S*)-(((2*R*,3*R*,4*R*,5*R*)-5-(2,4-dioxo-3,4-dihydropyrimidin-1(2*H*)-yl)-3-hydroxy-4-methyl-4-fluoro-tetrahydrofuran-2-yl)methoxy)-(phenoxy)-phosphorylamino)-propanoate (2.2). (52 %). LC-MS (ESI) 528 $(M+H)^+$. $^1H$ NMR (DMSO-$d_6$, 400 MHz) $\delta$ 11.24 (br.s., 1H), 7.56 (d, *J* = 8.1 Hz, 1H), 7.38 (m, 2H), 7.20 (m, 3H), 6.05 (m, 2H), 5.86 (m, 1H), 5.54 (d, *J* = 8.1 Hz, 1H), 4.36 (m, 1H), 4.23 (m, 1H), 4.02 (m, 2H), 3.82 (m, 2H), 1.25 (d, *J* = 22.2 Hz, 3H), 1.21 (d, *J* = 6.6 Hz, 3H), 0.66 (m, 2H), 0.57 (m, 2H).

**Example 5.** Preparation of a pharmaceutical composition in the form of tablet.

Starch (1600 mg), ground lactose (1600 mg), talk (400 mg), and 1000 mg of prodrug 1A.1, 1B.4, 1B.5, 1B.6 or 1B.7 were mixed together and pressed into bar. The resulting bar was comminuted into granules and sifted through a sieve to collect granules of 14-16 mesh. The granules thus obtained were shaped into tablets of suitable form weighing 300 or 600 mg each.

**Example 6.** Preparation of a pharmaceutical composition in the form of capsules.

Prodrug 1A.1, 1B.4, 1B.5, 1B.6 or 1B.7 was carefully mixed with a lactose powder in a ratio of 2:1. The resulting powdery mixture was packed into gelatin capsules of suitable size with either 300 or 600 mg in each capsule.

**Example 7.** Preparation of a pharmaceutical composition in the form of compositions for intramuscular, intraperitoneal, or subcutaneous injections.

A prodrug of formula 1A.1, 1B.4, 1B.5, 1B.6 or 1B.7 (500 mg) was mixed with chlorobutanol (300 mg), propylene glycol (2 ml), and injectable water. The resulting solution was filtered, placed into 5 ml ampoules, and sealed.

**Example 8.** Assessment of the metabolic parameters of prodrugs 1A.1 and 1A.2 and the TAF prototype in human peripheral blood mononuclear cells (PBMCs).

Generic solutions of tested compounds of formulas 1A.1, 1A.2 and TAF were prepared in DMSO (Sigma) and kept at -20°C. The PBMCs (kept in liquid nitrogen before use) were extracted from human blood by means of Ficoll-Paque Premium (GE Healthcare) gradient centrifugation. The PBMCs were placed in 24-well plates (Greiner Bio-one), 1.5 mln cells per well (4.2 mln/ml), in the RPMI-1640 medium containing L-glutamine (2 mM), sodium pyruvate (0.11 mg/ml), essential and nonessential amino acids, penicillin 50 Un/ml, streptomycin (50 $\mu$g/ml) (all reagents acquired from PanEco), and 5% HI (Heat Inactivated) fetal bovine serum (HyClone). The cells were incubated overnight at 37°C in an atmosphere of 5% $CO_2$. The next day, tested and reference compounds in a final concentration of 30 $\mu$M were added to the cells. The cells and compounds were incubated at 37°C in an atmosphere of 5% CO2. After 2, 4, 8, 24, 48, and

72 hours of incubation, nonadherent cells were together with the medium transferred into 1.5 ml test tubes (Eppendorf) and centrifuged for 5 minutes at 1000 g to remove the medium. The cells were washed with 1 ml of a phosphate buffer (Gibco) and lysed with 200 μl of 70% methanol cooled to -20°C. The cells that were adherent to the wells were washed with 1 ml of PBS (Gibco) and lysed with 200 μl of 70% methanol cooled to -20°C. The lysates of adherent and nonadherent cells from respective wells were combined and stirred.

The content of tenofovir (TFV) and diphosphate tenofovir (DP-TFV) in the cell lysates was determined by UPLC-MS/MS using a 1290 UPLC System (Agilent) chromatograph and a QTrap5500 System (AB Sciex) mass spectrometer with a triple quadrupole. The analytes were separated on a Thermo Hypercarb (50×3.0 mm, 5 μm, Thermo Scientific) column in a mobile phase comprising A - 0.5% ammonia in 25 mM of ammonium acetate and B - 0.5% ammonia in 25 mM of ammonium acetate: 2-propanol: methanol (1:1:3) at a flow rate of 0.8 ml/min. Electrospraying (TurbolonSpray) in the negative ion mode was used as an ion source. Analytes were detected in an MRM mode with the following transitions for TFV: 286>107, 286>79, 286>63 m/z and for DP-TFV: 446>348, 446>176, 446>158, 446>79 m/z. Chromatograms were analyzed using the Analyst 1.5.2 Software (AB Sciex). The concentrations of TFV and diphosphate TFV in cell lysates were estimated from calibration curves obtained using reference samples of TFV and DP-TFV in 70% methanol. The results are given in Table 1.

**Example 9.** Evaluation of anti-HIV activity of the prodrugs of general formula 1 and the prototype (TAF).

The antiviral activity of tested compounds was evaluated on the T-lymphocytes line, SupT1. The cells were infected with the HIV strain NL4.3 carrying a gene encoding the green fluorescent protein (NL4.3-GFP). A virus preparation was obtained by means of transfection of the 293T cells of antiviral DNA. After 48 hours of transfection, the medication was frozen and stored until being used. To increase the efficiency of infection, the suspension of SupT1 cells was sedimented from the infection mixture by centrifugation. Tested compounds were added to the cells immediately before adding the virus. After 2 hours of incubation, the infection mixture was replaced by a fresh culture medium with tested compounds. The efficiency of infection was evaluated after 45 hours by computing the percentage of fluorescence-bright cells against uninfected cell cultures. Concurrently, the cytotoxicity of tested compounds was evaluated in the same, but uninfected, cell line SupT1 using the XTT reagent. To determine antiviral activity and cytotoxicity, serial tenfold dilutions of the preparations were used (starting with 10 μM for antiviral activity and from 100 μM for cytotoxicity). DMSO (0.1%) was used for negative control. The values of $EC_{50}$, $CC_{50}$ and SI (selectivity index) were found. The quality of tests was evaluated based on the following controls: signal to background ratio, integrase inhibitor raltegravir (1 μM), and reproducibility of test results. Emetine (0.03, 0.09, and 0.2 μM) was used as the reference for cytotoxicity evaluation. The results are summarized in Table 2.

**Example 10.** Evaluation of anti-HCV activity and cytotoxicity for the prodrug of formula 1B.7, the compound of formula 2.2, and Sovaldi®.

The antiviral activity of tested compositions comprising prodrugs was evaluated using an Huh7 human hepatocellular carcinoma cell line stably transfected with an HCV replicon. A cell suspension in a complete culture medium (DMEM 1X, Cellgro; cat. # 10-013-CV) was transferred into 96-well plates (50 μl per well) with a final density of 7500 cells per well. The serial dilutions of tested prodrugs were prepared from a fresh 200-fold generic solution in DMSO with 11 concentration points dilution for each step, starting from 20 nM in a complete medium and were used as 2-fold solutions in two replicates. Minimum 4 hours after planting the cells, 50 μl of serial prodrug dilutions was added to each well. The final prodrug concentration varied from 10 nM to 0.1 pM and that of DMSO, 0.5%. The plate with cells was incubated for 3 days at 37°C under humidified 5% $CO_2$. Upon completion of incubation, the medium was removed by turning the plate over and shaking it carefully. The cells were fixed for one minute with 100 μl of a 1:1 acetone:methanol solution, washed 3 times with a phosphate buffer (PBS), and blocked for 1 h at room temperature using a 10% fetal bovine serum (FBS) in PBS (150 μl/well). The cells were washed 3 times with PBS and incubated for 2 h at 37°C with anti-NS5B HCV antibodies (100 μl/well) using Affinity BioReagents (cat. # MA1-080) and diluting the generic solution (1 mg/ml) in a ratio of 1:4000 in 10% FBS-PBS. The cells were washed 3 times with PBS and developed by an OPD solution (100 μl/well) using for each plate one OPD tablet dissolved in a citrate/phosphate buffer (12 ml), whereto 5 μl of 30% $H_2O_2$ was added, for 30 minutes in the dark at room temperature. The reaction was stopped by 2N $H_2SO_4$ (100 μl/well), and OD490 was measured using the multifunctional reader Victor$^3$ V 1420 (Perkin Elmer). The values of $EC_{50}$ for tested prodrugs were measured from an activity curve plotted using the GraphPad Prizm software. The new prodrug of formula 1B.7 has against the 1b (gT1b) HCV genotype $EC_{50}$ = 15.0-27.0 nM and $EC_{90}$ = 128.0 nM; Sovaldi®, $EC_{50}$ = 45-170 nM and $EC_{90}$ = 590 nM; the cyclohexyl ester of formula 2.1, $EC_{90}$ = 250.0 nM, and the cyclopropyl ester of formula 2.2, $EC_{90}$ = 73.0 nM and $EC_{90}$ = 410.0 nM (Table 3). Consequently, the new prodrug of formula 1B.7 is more than three times more active than Sovaldi®, two times more active than the compound of formula 2.1, and more than three times more active than the compound of formula 2.2. The results obtained are summarized in Table 3.

The cytotoxicity of tested compositions comprising prodrugs was evaluated concurrently in the same cell line Huh7 using an ATPLite kit (Perkin-Elmer, Boston, USA) in accordance with the manufacturer's instruction. The cell suspension in a complete culture medium (DMEM 1X, Cellgro; κaT. # 10-013-CV) was transferred into 96-well plates with black walls and transparent bottoms (50 μl/well) with a final density of 7500 cells per well. Eighteen hours after planting the cells, solutions for serial drug dilution (50 μl/well) were added. The plate with cells was incubated for 4 days at 37°C in a humidified 5% $CO_2$ atmosphere. The cells were then twice washed with PBS (200 μl/well) and lysed by adding a lytic buffer (50 μl/well); all reagents were taken from the ATPLite kit. Following a 5-minute stirring on a shaker, a substrate was added (50 μl/well). After an additional 5-minute incubation, the plate was kept for 10 minutes in the dark, and luminescence in the wells was measured using the multifunctional reader Victor$^3$ V 1420 (Perkin Elmer). The values of $CC_{50}$ for tested prodrugs were measured from a cytotoxicity curve plotted using the GraphPad Prizm software. In particular, for the new prodrug of formula 1 B.7 cytotoxicity was found to be $CC_{50}$ > 100 μM (Table 3) and the therapeutic window (therapeutic index TI = $EC_{50}/CC_{50}$), TI > 6 000.0.

**Example 11.** Evaluation of kinetic solubility for compounds.

*Principle of the method.* The tested compound was dissolved in DMSO to reach a 10-mM concentration and then poured into an aqueous solvent (a phosphate buffer, water, or universal buffers with different pH values) to bring the concentration down to 200 μM. The resulting solution placed in a 96-well filter plate (Millipore's MultiScreen Solubility Filter Plate) was incubated for an hour at room temperature on a shaker, and the residue was filtered off *in vacuo.* The absorption spectrum of the compound was recorded on a spectrophotometer in a range of 240-400 nm with a 10-nm increment. For quantitative estimation of solubility, a calibration curve of standard solutions (0 - 200 μM) comprising 40% of acetonitrile was used. The range of estimated concentrations was 3-200 μM. The test procedure was performed in duplicate.

*Preparation of calibration standards.* Calibration standards were prepared from 50-fold stock solutions in DMSO diluted in a buffer with 40% acetonitrile, which was added to ensure complete solubility of the tested compound in the calibration sample. Six standard samples with concentration of 0, 3.125, 12.5, 50, 100, and 200 μM were prepared in the wells of a 96-well UV plate by adding 4 μl of corresponding 50-fold stock solutions in DMSO to 196 μl of a buffer comprising 40% of acetonitrile. The concentration of DMSO in all points was constant and equal to 2% (v/v).

To plot calibration curves, the optical spectrum of the UV plate was recorded in a wavelength range of 250-400 nm with a 10-nm increment. Based on the spectral data for each compound, wavelengths were selected to meet the following criteria:

- For minimum compound concentration, OD > 0.1 (AU);
- For maximum compound concentration, OD < 2.0.

For each compound, a calibration curve was plotted with OD at selected wavelength as a function of concentration.

*Evaluation of kinetic solubility for compounds.* Solubility was evaluated in a MultiScreen Solubility (Millipore Corp.) filter plate as follows:

- Into each well of the MultiScreen Solubility filter plate, 196 μl of a buffer (without acetonitrile) and 4 μl of a 10-mM compound in DMSO or 4 μl of DMSO (for a blank matrix) were added. The plate was incubated for one hour on a shaker (400 rev/min) at room temperature.
- The resulting solutions were filtered off through the filter plate by means of vacuum (10" Hg) into a polypropylene plate with a U-shaped bottom.
- From the U-bottom plate, the filtrate (120 μl/well) was transferred into a new UV plate, whereto acetonitrile (80 μl/well) was added.
- The optical density of resulting solutionoB for a pre-selected wavelength was measured for each compound.

*Calculations.* The final concentration of a compound in the filtrate was computed as follows:

$$C_{filtrate} = (OD_{\lambda}Filtrate - OD_{\lambda}Blank)/Slope \times 1.67,$$

wherein:

$OD_{\lambda}$Filtrate is the optical density of filtrate for a selected wavelength;
$OD_{\lambda}$Blank is OD of a blank matrix;

Slope is the gradient of the calibration line;
1.67 is dilution factor for the filtrate diluted with acetonitrile.

The findings are presented in Table 6.

**Example 12.** X-ray powder phase analysis of prodrug samples.

All diffraction patterns were recorded on a Bruker D8 Advance Vario diffractometer equipped with a copper-anode X-ray tube, Ge(111) monochromator (CuKo^) and LynxEye position-sensitive detector, in peek-a-boo settings. The shot range was 3-90° ***26*** for sample s5 and 5.7-90° ***26*** for the other samples, and the increment was 0.01° ***26***. The analysis was carried out using the Bruker Topas5 software ['Bruker TOPAS 5 User Manual. - Karlsruhe, Germany : Bruker AXS GmbH, 2015.].

Samples were obtained by recrystallization of the compound of formula 1B.7 from a mixture of ethyl acetate with methyl-*tert*-butyl ester (1:1), ethanol, ethyl acetate, and a mixture of acetic acid with water had polycrystalline forms. X-ray powder phase analyses of these samples revealed a similarity of their qualitative phase structures and an insignificant difference in their phase relations. The samples had an orthorhombic phase with the following unit cell parameters: ***a*** = 28.1056(8) A, ***b*** = 16.8998(4) A, and ***c*** = 5.25380(12) A. Systematic extinction analysis allows to assume a spatial group of $P2_12_12_1$. A unit cell volume of 2495.45(11) $A^3$ corresponds to the claimed composition and Z' = 1. The samples also have a monoclinic phase with the following unit cell parameters: ***a*** = 16.2770(6) A, ***b*** = 16.9117(8) A, ***c*** = 5.20429(15) A, $\beta$= 117.822(2)°. Systematic extinction analysis allows one to assume a spatial group of $P2_1$. A unit cell volume of 1266.98(9) $A^3$ corresponds to the claimed composition and Z' = 1. The evaluation of phase relations based on comparisons of integral peak intensities suggests that the content of monoclinic phase varies from 30 to 50%.

The sample obtained by recrystallization of the compound of formula 1B.7 from a mixture of dimethyl sulfoxide with water is a white substance of crystalline form. According to X-ray powder analysis data, the sample of said form is single-phase and consists of an orthorhombic phase with the following unit cell parameters: ***a*** = 28.1056(8) A, ***b*** = 16.8998(4) A, ***c*** = 5.25380(12) A. Systematic extinction analysis allows one to assume a spatial group of $P2_12_12_1$. A unit cell volume of 2495.45(11) $A^3$ corresponds to the claimed composition and Z' = 1.

**Example 13.** Evaluation of stability in biological matrix for compositions comprising prodrug 1B.7.

Initial compositions comprising the prodrug of formula 1B.7 (the tested compounds) were prepared with a concentration of 10 mM in DMSO. From said compositions, 100-fold working solutions were prepared with a concentration of 100 $\mu$M in a mixture of acetonitrile : water with a volumetric ratio of 1:1.

a) Stability in S9 fraction. The reaction mixture was prepared in a 0.1 M potassium phosphate buffer (pH 7.4 BD Gentest) in a total final volume of 250 $\mu$l and contained a 1 mM NADPH-tetrasodium salt (AppliChem), 7 mM glucose-6-phosphate sodium salt (Sigma), 1.5 U/ml glucose-6-phosphate dehydrogenase (Sigma), 3.3 mM $MgCl_2$ (Sigma), 5 mM uridine-5-diphosphate-glucuronic acid trisodium salt (UDP-GlcA, Sigma), and 1 $\mu$M tested compound (final concentrations). The metabolic reaction was initiated by adding a suspension of human liver S9 fraction (BD Gentest), and the final concentration of protein was 1 mg/ml. The reaction mixture was incubated at 37°C on a Vortemp56 shaker with stirring at 400 rev/min. After certain intervals (0, 0.25, 0.5, 1, 2, 4, 6, 8, 24 h), a 30 $\mu$l sample was taken; the reaction was stopped by adding cold acetonitrile (180 $\mu$l) comprising an internal standard to the sample taken. Proteins were deposited on ice for 15 min. The samples were then centrifuged, and the supernatant (150 $\mu$l) was sampled for analysis for 10 min at 3000 rev/min. The incubation was performed in two replicates, with each sample measured twice.

b) Stability in artificial gastric and intestinal juices. The tested composition comprising the prodrug of formula 1B.7 in a final concentration of 1 $\mu$M was incubated in artificial gastric juice (0.2% NaCl in 0.7% v/v HCl) and artificial intestinal juice (0.05M $KH_2PO_4$, pH 6.75). The incubation was performed in a Vortemp56 shaking incubator at 37°C with stirring at a rate of 300 rev/min. After certain intervals (0, 0.25, 0.5, 1, 2, 4, 6, 8, 24 h), a 30 $\mu$l sample was taken; the reaction was stopped by adding cold acetonitrile (180 $\mu$l) comprising an internal standard to the sample taken. The samples were then centrifuged, and the supernatant (150 $\mu$l) was sampled for analysis for 10 min at 3000 rev/min. The incubation was performed in two replicates, with each sample measured twice.

c) Stability in blood plasma. The tested compound in a final concentration of 1 $\mu$M was incubated in pooled human blood plasma (Innovative Research). Incubation was performed in a Vortemp56 shaking incubator at 37°C with stirring at a rate of 300 rev/min. After certain intervals (0, 0.25, 0.5, 1, 2, 4, 6, 8, 24 h), a 30 $\mu$l sample was taken; the reaction was stopped by adding cold acetonitrile (180 $\mu$l) comprising an internal standard to the sample taken.

The samples were then centrifuged, and the supernatant (150 μl) was sampled for analysis for 10 min at 3000 rev/min. The incubation was performed in two replicates, with each sample measured twice.

*Sample analysis.* Samples were analyzed using an HPLC-MS/MS technique developed for each tested prodrug, wherein the chromatographic system 1290 Infinity II (Agilent Technologies) was combined with the tandem mass spectrometer QTRAP5500 (AB Sciex). When developing conditions for mass-spectrometric detection, the solutions of tested compounds in a mixture of acetonitrile-water 1:1 with a concentration of 100 ng/ml were injected directly into the mass spectrometer using a syringe pump with electrospray ionization in a positive ion mode. Scanning in a total ion current mode (MS1) allowed us to identify a molecular ion for each compound, and basic product ions were recorded in MS2 mode. Then, to attain maximum sensitivity, the MS/MS technique was optimized in MRM mode. In quantitative chromatogram processing, the most intensive MRM transition was used for the analyte and the internal standard. Separation was carried out by means of linear gradient elution chromatography on a YMC Triart C18 column (50 x 2 mm, 1.9 μm) in a mobile phase consisting of 0.1% formic acid in water and 0.1% formic acid in acetonitrile. Tolbutamide (Fluka) was used as the internal standard.

*Computations.* Half-life ($T_{1/2}$) was found from the kinetics of tested prodrug elimination in an antiviral composition during incubation in a biological matrix. The computations were based on the values of chromatographic peak areas for compounds in test samples normalized to the internal standard signal. From linear dependence of log normalized areas of chromatographic peaks on time, the constant of elimination rate was calculated (k is linear section slope). Then, half-life was found: $T_{1/2} = 0.693 / k$. It was discovered, in particular (see Table 4), that the prodrug of formula 1B.7, the compound of formula 2.1, and Sovaldi® have comparable stabilities in human gastric juice ($T_{1/2}^{SGF}$ = 12.7 h - 17 h), in human intestinal juice ($T_{1/2}^{SIF}$ > 20 h), and in human plasma ($T_{1/2}^{HPL}$ > 24 h). At the same time, the prodrug of formula 1B.7 more actively metabolizes in human hepatic microsomal S9 fraction and has half-life $T_{1/2}^{HS9}$ = 0.05 h, while its prototype Sovaldi®has $T_{1/2}^{HS9}$ = 0.57 h and cyclohexyl ester of formula 2.1, $T_{1/2}^{HS9}$ = 1.4 h (Table 4), which means that the prodrug of formula 1B.7 metabolizes in human hepatic microsomal S9 fraction 11 times faster than Sovaldi® and 28 times faster than the compound of formula 2.1. The results obtained are summarized in Table 4.

**Example 14.** Pharmacokinetic (PK) study of compositions comprising the prodrug of formula 1B.7 and Sovaldi® in the rat liver.

*Preparation of compositions comprising the prodrug* of formula *1B.7* and *Sovaldi*® *for administration to rats.* The tested composition was administered at a dose of 50 mg/kg. To this end, compositions were prepared with a concentration of prodrug 1B.7 or Sovaldi® of 5.0 mg/ml in a 0.5% solution of hydroxypropylmethylcellulose (HPMC), to which 5% ethanol was added, as follows: to a weighed portion of the prodrug of formula 1B.7 or Sovaldi®, an appropriate amount of HPMC was added, and the mixture was triturated dry in a mortar, whereafter a proper quantity of 5% ethanol in distilled water was gradually added portionwise, and the mixture was carefully stirred to obtain a suspension suitable for intragastric administration.

*Administration of compositions comprising the prodrug* of formula *1B.7* and *Sovaldi*® *to animals. Preparation of blood plasma and liver samples.* The study was carried out on Sprague Dawley rats. The rats were divided into groups of 6 based on selected time points (1, 2, 4, 6, 8, 10, 12, 16 and 24 h). The rats were weighed, and the volume of compositions comprising the prodrug of formula 1B.7 or Sovaldi® was calculated for each animal at a rate of 10 ml/kg. Compositions comprising the prodrug of formula 1B.7 or Sovaldi® were administered intragastrically through a feeding tube. In the intervals between administrations to the animals of a certain group, samples of liver and blood were taken. Some time after the administration, the rat was euthanized by $CO_2$ inhalation. Immediately after the euthanasia, the animal was quickly opened up, and its upper lobe of the liver was instantaneously placed in liquid nitrogen. The frozen liver fragment was then transferred into a labeled test tube cooled with liquid nitrogen. The samples were kept in liquid nitrogen till the end of the experiment and then put into an ultra-cold freezer at -80°C.

*Sample preparation.* A liver sample weighing about 1 g was triturated in a mortar while being cooled with liquid nitrogen. The resulting powder was poured over with triple-volume methanol with 70% EDTA methanol and was twice homogenized for 45 s (with a 10-second interval) at a rate of 6.3 m/s using the Omni Bead Ruptor 24 homogenizer. To 360 μl of thus obtained homogenate, 40 μl of ten-fold standard solution comprising PSI-7409 and H027-4261 (or methanol, in case of experimental samples) and 100 μl of internal standard solution (5-bromouridine triphosphate) with a concentration of 25 ng/ml were added. After stirring and centrifuging, 400 μl of supernatant was diluted with 400 μl of a 1% formic acid solution in a mixture of methanol - water (1:1). Then, solid-phase extraction was performed using Waters Oasis WAX cartridges. The resulting product was eluted with 800 μl of a 5% solution of ammonia in methanol, and the eluate was evaporated and redissolved in 200 μl of methanol.

*HPLC-MS/MS analytical conditions.* Samples were analyzed using an HPLC-MS/MS technique, wherein the HPLC system Agilent 1290 Infinity II was combined with the AB Sciex QTrap 5500 mass spectrometer. Separation was carried out on a Thermo Hypercarb column (50 x 3 mm, 5 μm). A 25-mM solution of ammonium acetate with 0.5%

ammonium was used as mobile phase A (MPA); a 25-mM solution of ammonium acetate in a mixture of water-isopropanol-acetonitrile (1:1:3) with 0.5% ammonium was used as mobile phase B (MPB). Separation was performed in gradient mode: 0-0.3 min - 5% MPB; 3-3.4 min - 50% MPB; 3.6-4.5 min - 5% MPB. PSI-7409 and H027-4261 were recorded in MRM mode with ion transitions of 499/159 and 410/150, respectively.

*Pharmacokinetic analysis.* Pharmacokinetic analysis of "liver concentration versus time" data was performed by a non-compartmental technique using Phoenix™ WinNonlin® 6.3 (Pharsight Corp.) and GraphPad Prizm software. The following pharmacokinetic parameters were computed: maximum concentration in liver ($C_{max}$) and time of achievement thereof ($T_{max}$), half-life ($T_{1/2}$), and area under the PK curve ($AUC_{0-t}$, $AUC_{0-inf}$). The findings are represented in Table 5. As can be seen from Table 5, the concentration and $AUC_{24h}$ of triphosphate PSI-7409 resulting from the metabolism of prodrug 1B.7 in the rat liver are $C_{max}$ =3224.0 ng/g and $AUC_{24h}$ = 30487.0 ngh/g, respectively, while Sovaldi® exhibiting similar metabolism has $C_{max}$ =1,934.0 ng/g and $AUC_{24h}$ = 16,796.0 ng.h/g, and cyclohexyl ester of formula 2.1 has $C_{max}$ = 557 ng/g and $AUC_{24h}$ = 6,484.0 ng.h/g (Table 5). This suggests that the new prodrug of formula 1B.7 is almost twice more effective in its liver metabolism into target triphosphate PSI-7409 (drug), as compared to Sovaldi® and 4.7 times, as compared to cyclohexyl ester of formula 2.1. The findings are represented in Table 5.

**Industrial applicability**

The invention could be used in medicine and veterinary.

**Claims**

1. A prodrug of general formula 1, a stereoisomer thereof, and an isotopically enriched analog, a pharmaceutically acceptable salt, a hydrate, a solvate, crystalline or polycrystalline forms of the prodrug of general formula 1 or a stereoisomer thereof,

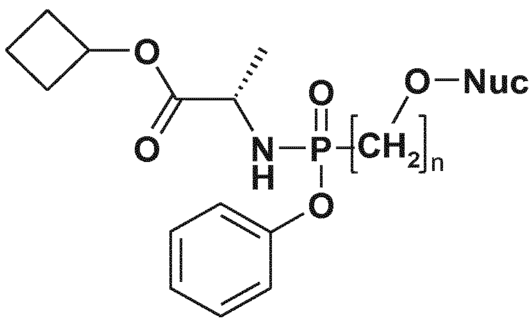

1

wherein:

n is 1 or 0;
Nuc is

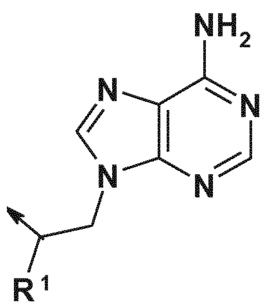

or

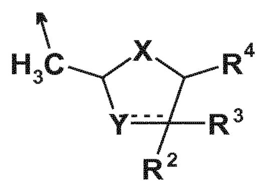

,

$R^1$ is hydrogen or methyl;
$R^2$, $R^3$ are optionally identical substituents selected from H, F, Cl, $CH_3$, OH provided that a solid line together with a dashed line above thereof (----) denote a carbon-carbon single bond (C-C), or $R^2$ and $R^3$ refer to hydrogen

provided that a solid line together with a dashed line above thereof (----) denote a carbon-carbon double bond (C=C);

$R^4$ is a substitute selected from $R^{4.1}$- $R^{4.5}$:

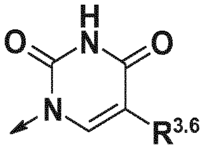
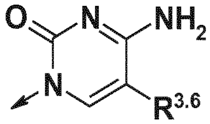
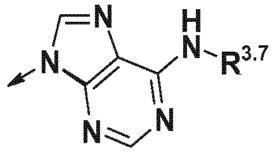
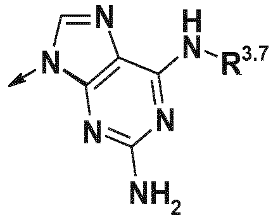
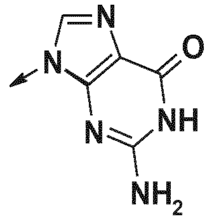

$R^{4.1}$ $R^{4.2}$ $R^{4.3}$ $R^{4.4}$ $R^{4.5}$

$R^{3.6}$ is a substitute selected from H, F, Cl, $CH_3$, or $CF_3$;

$R^{3.7}$ is hydrogen, $C_1$-$C_4$-alkyl, or $C_3$-$C_6$-cycloalkyl;

X is O, $CH_2$, or C=$CH_2$;

Y is O, S, $CH_2$, or a HO-CH group provided that a solid line together with a dashed line above thereof (----) denote a carbon-carbon single bond (C-C), or Y is a CH group provided that a solid line together with a dashed line above thereof (----) denote a carbon-carbon double bond (C=C).

2. The prodrug according to Claim 1, which is a compound of general formula 1A or 1B, their stereoisomer, and an isotopically enriched analog, a pharmaceutically acceptable salt, a hydrate, a solvate, and crystalline or polymorphic forms of the compound of general formula 1A or 1B, or a stereoisomer thereof,

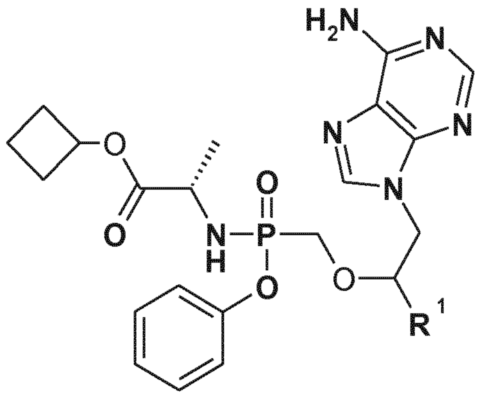

1A

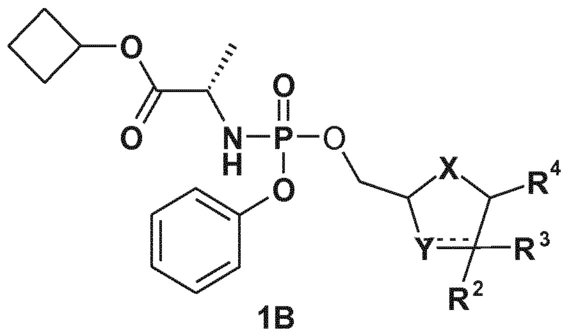

1B

wherein a solid line together with a dashed line above (----), $R^1$, $R^2$, $R^3$, $R^4$, X, and Y are as defined above.

3. The prodrug according to Claim 1 selected from (*S*)-cyclobutyl 2-((*S*)-(((*R*)-1-(6-amino-9*H*-purin-9-yl)propan-2-yloxy)methyl)(phenoxy)phosphorylamino)-propanoate (1A.1),

(*S*)-cyclobutyl 2-((*R*)-(((*R*)-1-(6-amino-9*H*-purin-9-yl)propan-2-yloxy)methyl) (phenoxy)phosphorylamino)-propanoate (1A.2),

(*S*)-cyclobutyl 2-{(*S*)-[(2*S*,3*R*,5*S*)-3-hydroxy-5-(5-methyl-3,4-dihydro-2,4-dioxo-2H-pyrimidin-1-yl)-tetrahydrofuran-2-ylmethoxy]-phenoxy-phosphorylamino}-propanoate (1B.1),

(*S*)-cyclobutyl 2-{(*S*)-[(2*S*,3*S*,4*R*,5*S*)-3-hydroxy-4-fluoro-5-(5-methyl-3,4-dihydro-2,4-dioxo-2*H*-pyrimidin-1-yl)-tetrahydrofuran-2-ylmethoxy]-phenoxy-phosphorylamino}-propanoate (1B.2),

(*S*)-cyclobutyl 2-{(*S*)-[(2*R*,3*R*,5*R*)-5-(4-amino-2-oxo-2*H*-pyrimidin-1-yl)-3-hydroxy-4,4-difluoro-tetrahydrofuran-2-ylmethoxy]-phenoxy-phosphorylamino}-propanoate (1B.3),

(*S*)-cyclobutyl 2-{(*S*)-[(1*R*,3*S*,5*S*)-3-(2-amino-6-oxo-1,6-dihydro-purin-9-yl)-5-hydroxy-2-methylene-cyclopentylmethoxy]-phenoxy-phosphorylamino}-propanoate (1B.4),

(*S*)-cyclobutyl 2-{(*S*)-[(2*R*,5*S*)-5-(4-amino-2-oxo-2*H*-pyrimidin-1-yl)-[1,3]oxatiolan-2-ylmethoxy]-phenoxy-phosphorylamino-propanoate (1B.5),

(*S*)-cyclobutyl 2-{(*S*)-[(2*R*,5*S*)-5-(4-amino-5-fluoro-2-oxo-2*H*-pyrimidin-1-yl)-[1,3]oxatiolan-2-ylmethoxy]-phenoxy-phosphorylamino}-propanoate (1B.6),

(*S*)-cyclobutyl 2-{(*S*)-[(2*R*,3*R*,4*R*,5*R*)-5-(3,4-dihydro-2,4-dioxo-2*H*-pyrimidin-1-yl)-3-hydroxy-4-methyl-4-fluoro-tetrahydrofuran-2-ylmethoxy]-phenoxy-phosphorylamino}-propanoate (1B.7),

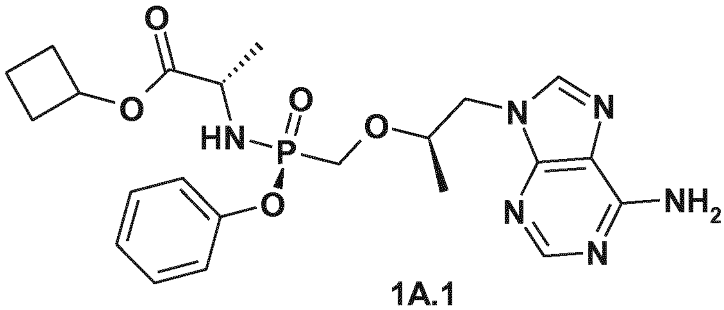

1A.1

,

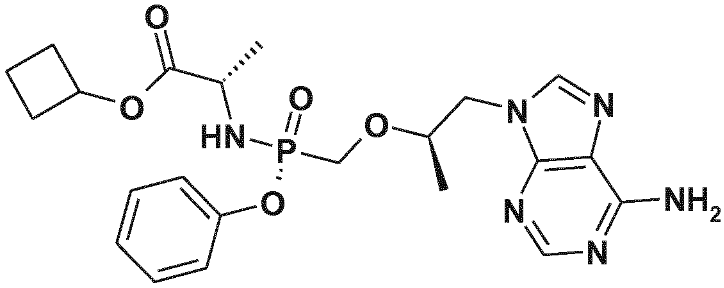

1A.2

,

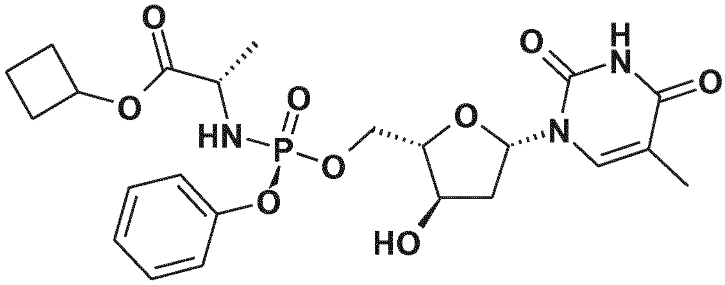

1B.1

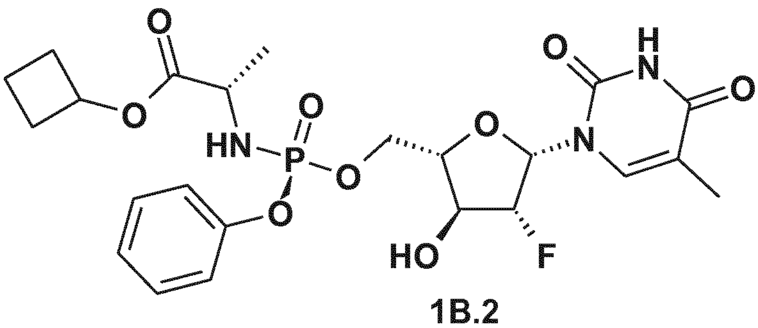

1B.2

,

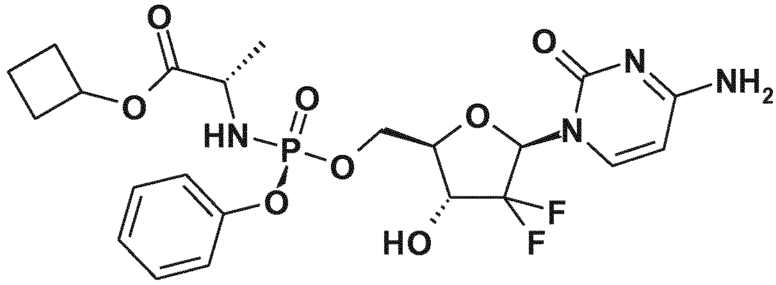

1B.3

,

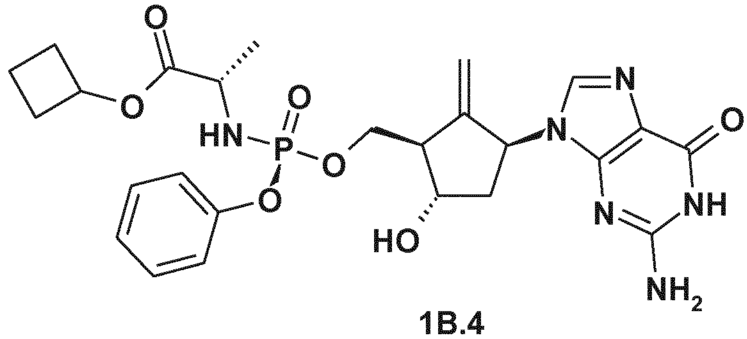

1B.4

,

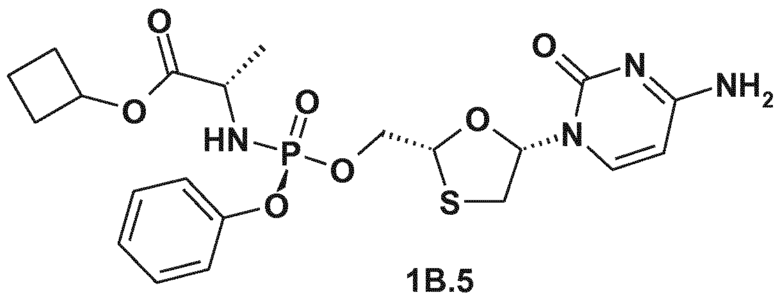

1B.5

,

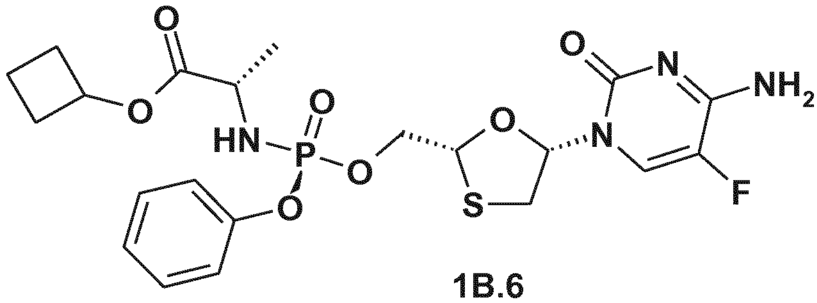

1B.6

,

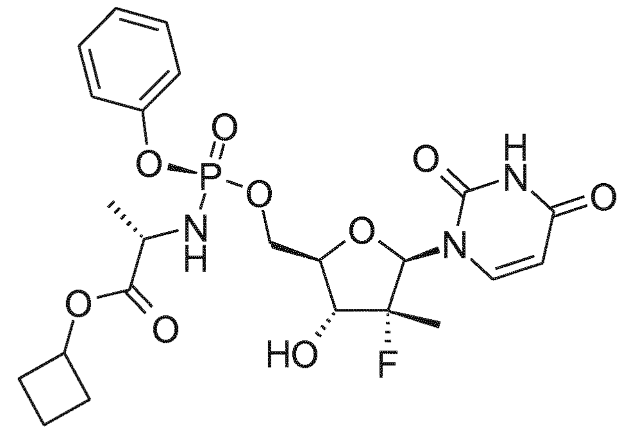

**1B.7**

,

a stereoisomer thereof, and their isotopically enriched analog, pharmaceutically acceptable salt, hydrate, solvate, or crystalline or polycrystalline forms.

4. A pharmaceutical composition comprising a therapeutically effective amount of the prodrugs according to Claims 1-3 and a pharmaceutically acceptable carrier.

5. A method for the combination therapy of viral and cancer diseases in subjects in need thereof, said method involving simultaneous or successive administration of a therapeutically effective amount of the prodrug of general formula 1, or a stereoisomer thereof, or an isotopically enriched analog, a pharmaceutically acceptable salt, a hydrate, a solvate, or crystalline or polycrystalline forms of the prodrug of general formula 1 or its stereoisomer, or the pharmaceutical composition according to Claim 4 and another antiviral or anticancer agent.

**INTERNATIONAL SEARCH REPORT**

International application No.
PCT/RU 2017/000209

A. CLASSIFICATION OF SUBJECT MATTER
see supplemental sheet

According to International Patent Classification (IPC) or to both national classification and IPC

B. FIELDS SEARCHED

Minimum documentation searched (classification system followed by classification symbols)
C07H 19/10, 19/20, C07F 9/44, 9/6561, A61K 31/7076, 31/7072, 31/708, 31/664, 31/675, A61P 31/18, 31/20

Documentation searched other than minimum documentation to the extent that such documents are included in the fields searched

Electronic data base consulted during the international search (name of data base and, where practicable, search terms used)
RUPAT, EAPO, Esp@senet, USPTO, CIPO, DEPATISnet, PCT Online, SIPO, DWPI

C. DOCUMENTS CONSIDERED TO BE RELEVANT

| Category* | Citation of document, with indication, where appropriate, of the relevant passages | Relevant to claim No. |
|---|---|---|
| X | CN 106317116 A (ZHANG HONGLI et al.) 11.01.2017, abstract, p. 4, line 2, 2-oe compound, paragraph [0035]-[0037], [0067], col. 1, last compound examples, claims 3, 7-10 | 1, 2, 4, 5 |
| X | WANG, P. et al. Phosphoramidate prodrags of (-)-β-D-(2R,4R)-dioxolane-thymine (DOT) as potent anti-HIV agents. Antiviral Chemistry & Chemotherapy, 2012, 22 (5), pp.217-238, in particular abstract, p. 226, example 73 | 1, 2 |
| X | WO 2000/018775 A1 ( UNIVERSITY COLLEGE CARDIFF CONSULTANTS LIMITED et al.) 06.04.2000, c. 61, lines 5-25, abstract, the claims 1, 10, p 123, lines 30-32, claims 11-21 | 1, 2, 4, 5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 October 2017 (09.10.2017) | 23 November 2017 (23.11.2017) |
| Name and mailing address of the ISA/ RU | Authorized officer |
| Facsimile No. | Telephone No. |

Form PCT/ISA/210 (second sheet) (April 2005)

**INTERNATIONAL SEARCH REPORT**

International application No.
PCT/RU 2017/000209

C (Continuation). DOCUMENTS CONSIDERED TO BE RELEVANT

| Category* | Citation of document, with indication, where appropriate, of the relevant passages | Relevant to claim No. |
|---|---|---|
| X | WO 2008/121634 A2( FHARMASSET INC. et al.) 09.10.2008, abstract, p. 695, compound 25, 28, 1, 2, compound 49, 55, 56, 58, claim 3 | 1-5 |
| A | ZENG DEBINE et al. Discovery of 2'α-C-Methyl-2'βC-fluoridinephosphoramidate Prodrags as Inhibitors of Hepatitis S Virus, ACS Medicinal Chemistry Letters, 2016, 7(12), pp. 1197-1201, abstract, p. 1,2, table 1, compound 17 | 1-5 |

Form PCT/ISA/210 (continuation of second sheet) (April 2005)

INTERNATIONAL SEARCH REPORT

International application No.
PCT/RU 2017/000209

A. CLASSIFICATION OF SUBJECT MATTER

***C07H 19/10*** *(2006.01)*
***C07H 19/20*** *(2006.01)*
***C07F9/36*** *(2006.01)*
***C07F9/44*** *(2006.01)*
***C07F9/6561*** *(2006.01)*
***A61K31/7076*** *(2006.01)*
***A61K 31/7072*** *(2006.01)*
***A61K 31/708*** *(2006.01)*
***A61K 31/664*** *(2006.01)*
***A61K 31/675*** *(2006.01)*
***A61P 31/20*** *(2006.01)*
***A61P 31/18*** *(2006.01)*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2002008241 A
- US 7390791 B
- WO 2013025788 A
- WO 2013116720 A
- US 9296769 B
- US 7964580 B
- US 8334270 B
- RU 2478104
- US 9428491 B2, A.V. Ivachtchenko
- US 14845333 B
- WO 2010144646 A
- WO 2012040923 A
- WO 2012041014 A
- WO 2015110048 A1
- WO 2014033617 A1

### Non-patent literature cited in the description


- Nucleosides and Nucleotides for the treatment of viral diseases. **M.J. SOFIA.** Annual Reports in Medicinal Chemistry. 2014, vol. 49, 221-247
- **L.P. JORDHEIM et al.** Advances in the development of nucleoside and nucleotide analogues for cancer and viral diseases. *Nat. Rev. Drug. Discov.,* June 2013, vol. 12 (6), 447-464
- Antiviral Agents 2013. Academic Press, 2013, vol. 67
- **L. P. JORDHEIM et al.** Advances in the development of nucleoside and nucleotide analogues for cancer and viral diseases. *Nal. Rev.,* 2013, vol. 12, 447-464
- **R. F. SHINAZI et al.** Pharmacology of current and promising nucleosides for the treatment of human immunodeficiency viruses. *J. Antiviral Res.,* 2006, vol. 71, 322-334
- **E. DE CLERCQ.** The nucleoside reverse transcriptase inhibitors, nonnucleoside reverse transcriptase inhibitors, and protease inhibitors in the treatment of HIV infections (AIDS). *Adva Pharmacol.,* 2013, vol. 67, 317-358
- **A.S. RAY ; M.W. FORDYCE ; M.J.M. HITCHCOCK.** Tenofovir alafenamide: A novel prodrug of tenofovir for the treatment of Human Immunodeficiency Virus. *Antiviral Research,* January 2016, vol. 125, 63-70, http://www.accessdata.fda.gov/drugsatfda-docs/nda/2015/ 207561Orig1s000PharmRpdf
- Nucleosides and Nucleotides for the treatment of viral diseases. **M.J. SOFIA.** Annual Reports in Medicinal Chemistry. 2014, vol. 49, 224
- **L. BONDADA et al.** Adenosine Dioxolane Nucleoside Phosphoramidates as Antiviral Agents for Human Immunodeficiency and Hepatitis B Viruses. *ACS Med. Chem. Lett.,* 2013, vol. 4, 747-751
- **D. GRIMM et al.** HBV life cycle and novel drug targets. *Hepatol. Int.,* 2011, vol. 5, 644-653
- **G. BORGIA ; I. GENTILE.** Treating chronic hepatitis B: today and tomorrow. *Curr. Med. Chem.,* 2006, vol. 13, 2839-2855
- **T. T. CHANG et al.** Long-term entecavir therapy results in the reversal of fibrosis/cirrhosis and continued histological improvement in patients with chronic hepatitis B. *Hepatology,* 2010, vol. 52, 886-893
- **P. MARCELLIN et al.** Regression of cirrhosis during treatment with tenofovir disoproxil fumarate for chronic hepatitis B: a 5-year open-label follow-up study. *Lancet,* 2013, vol. 381, 468-475
- **C.A. GENG et al.** Small-molecule inhibitors for the treatment of hepatitis B virus documented in patents. *Mini Rev. Med. Chem.,* 2013, vol. 13, 749-776
- **R.K. RAWAL et al.** 2'-Fluoro-6'-methylene-carbocyclic adenosine phosphoramidate (FMCAP) prodrug: In vitro anti-HBV activity against the lamivudine-entecavir resistant triple mutant and its mechanism of action. *Bioorg. Med. Chem. Lett.,* 2013, vol. 23, 503-506
- **G.R. BLUEMLING et al.** Targeted Delivery of Clevudine-5'-Monophosphate to the Liver After Oral Administration of a Clevudine-5'- Phosphoramidate Conjugate to Rats for the Treatment of HBV Infections. *Global Antiviral Journal,* 2015, vol. 11 (3), 97
- **M. SLUSARCZYK et al.** Application of ProTide Technology to Gemcitabine: A Successful Approach to Overcome the Key Cancer Resistance Mechanisms Leads to a New Agent (NUC-1031) in Clinical Development. *J. Med. Chem.,* 2014, vol. 57, 1531-1542
- **MESSINA, J. P.** Global Distribution and Prevalence of Hepatitis C Virus Genotypes. *Hepatology,* 2015, vol. 61 (1), 77-87

- **SOFIA, M. J. et al.** Discovery of a β-D-2'-Deoxy-2'-a-fluoro-2'-β-C-methyluridine (Sovaldi) Nucleotide Prodrug (PSI-7977) for the Treatment of Hepatitis C Virus. *J. Med. Chem.,* 2010, vol. 53, 7202-7218
- **I.M. JACOBSON et al.** Sofosbuvir for hepatitis C genotype 2 or 3 in patients without treatment options. *Engl. J. Med.,* 2013, vol. 368, 1867-1877
- **E. LEWIRZ et al.** Sofosbuvir for previously untreated chronic hepatitis C infection. *Engl. J. Med.,* 2013, vol. 368, 1878-1887
- **MURAKAMI et al.** Mechanism of activation of PSI-7851 and its diastereoisomer PSI-7977. *J. Biol. Chem.,* 2010, vol. 285 (45), 34337-34347
- **M. J. SOFIA et al.** Discovery of a β-D-20-Deoxy-20-r-fluoro-20-β-C-methyluridine Nucleotide Prodrug (PSI-7977) for the Treatment of Hepatitis C Virus. *J. Med. Chem.,* 2010, vol. 53, 7202-7218
- **W. CLUNG et al.** Discovery of PSI-353661, a Novel Purine Nucleotide. *ACS Med. Chem. Lett.,* 2011, vol. 2, 130-135
- **X.-J. ZHOU. et al.** Safety and Pharmacokinetics of IDX184, a Liver-Targeted Nucleotide Polymerase Inhibitor of Hepatitis C Virus, in Healthy Subjects. *Antimicrob. Agents Chegeneric.,* 2011, vol. 55, 76-81
- **J. LALEZARI et al.** Short-Term Monotherapy with IDX184, a Liver-Targeted Nucleotide Polymerase Inhibitor, in Patients with Chronic Hepatitis C Virus Infection. *Antimicrob. Agents Chegeneric.,* 2012, vol. 56, 6372-6378
- **C. MCGUIGAN et al.** Phosphorodiamidates as a Promising New Phosphate Prodrug Motif for Antiviral Drug Discovery: Application to Anti-HCV Agents. *J. Med. Chem.,* 2011, vol. 54, 8632-8645
- **J. H. VERNACHIO et al.** INX-08189, a phosphoramidate prodrug of 6-O-methyl-2'-C-methyl guanosine, is a potent inhibitor of hepatitis C virus replication with excellent pharmacokinetic and pharmacodynamic properties. *Antimicrob. Agents Chegeneric.,* 2011, vol. 55, 1843-1851
- **J. J. ARNOLD et al.** Sensitivity of Mitochondrial Transcription and Resistance of RNA Polymerase II Dependent Nuclear Transcription to Antiviral Ribonucleosides. *PLOS Pathog.,* 2012, vol. 8
- **P. WANG et al.** Phosphoramidate prodrugs of (-)-β-D-(2R,4R)-dioxolane-thymine (DOT) as potent anti-HIV agents. *Antiviral Chem. Chegenericapy,* 2012, vol. 22, 217-238
- **BUNGARD, H.** Design of Prodrugs. Elsevier, 1985, 7-9, 21-24
- **BERGE S.M. et al.** Pharmaceutical Salts. *J. Pharm. Sci.,* 1977, vol. 66, 1-19
- **M. J. SOFIA et al.** *J. Med. Chem.,* 2010, vol. 53, 7202-7218
- **C. GARDELLI et al.** Phosphoramidate Prodrugs of 20-C-Methylcytidine for Therapy of Hepatitis C Virus Infection. *J. Med. Chem.,* 2014, vol. 57, 2047-2057
- **COBURN C. A. et al.** *ChemMedChem.,* 2013, vol. 8, 1930-1940
- Bruker TOPAS 5 User Manual. Bruker AXS GmbH, 2015